# EUROPEAN PATENT SPECIFICATION

(11) **EP 1 699 446 B1**
(45) Date of publication and mention of the grant of the patent: **28.05.2008**
(21) Application number: 04814853.0
(22) Date of filing: 18.12.2004
(51) Int. Cl.: A61K 31/16

(54) **COMPOSITIONS AND METHODS FOR TREATING HEPATITIS C VIRUS (HCV) INFECTION**
ZUSAMMENSETZUNGEN UND VERFAHREN ZUR BEHANDLUNG VON HEPATITIS-C-VIRUS (HCV) INFEKTION
COMPOSITIONS ET PROCEDES POUR LE TRAITEMENT D'INFECTION PAR LE VIRUS DE L'HEPATITE C

(30) Priority: 19.12.2003 US 531543 P
(43) Date of publication of application: 13.09.2006
(73) Proprietor: Rigel Pharmaceuticals, Inc., South San Francisco, CA 94080 (US)
(72) Inventor: HOLSZTYNSKA, Elzbieta, J., Half Moon Bay, CA 94109-1872 (US); LO, Ray, San Leandro, CA 94579 (US); SUN, Thomas, W., Fremont, CA 94555 (US); WANG, Steven, X., Sunnyvale, CA 94087 (US)
(74) Representative: Mallalieu, Catherine Louise
(86) International application number: PCT/US2004/042717
(87) International publication number: WO 2005/063225

(56) References cited:
- WO-A-20/04018463
- US-A- 4 971 618
- BENCHARIT ET AT: "Crystal structure of human carboxyesterase 1 complexed with the Alzheimer's drug Tacrine; from binding promiscuity to seletive inhibition." CHEMISTRY AND BIOLOGY, vol. 10, 2003, - 2003 pages 341-349, XP001206040

## Description

### 2. TECHNICAL FIELD

The disclosure provides compositions for inhibiting HCV replication and/or proliferation. Further provided are methods of using the compositions as a therapeutic approach towards the treatment and/or prevention of HCV infection in animals and humans.

### 3. BACKGROUND

HCV is a single stranded RNA virus, which is the etiological agent identified in most cases of non-A, non-B hepatitis (NANBH), and is a common cause of acute sporadic hepatitis (Choo et al., Science 244:359 (1989); Kuo et al., Science 244:362 (1989); and Alter et al., in Current Perspective in Hepatology, p. 83 (1989)). Acute hepatitis C spontaneously resolves in only 20% of cases; the remaining 80% evolve towards chronic hepatitis C. The chronically infected patients show increased risk for chronic liver disease and cirrhosis. Persistent viral infection is believed to induce immune-mediated hepatocellular injury (*i.e.,* inflammatory liver disease), which frequently leads to the development of primary hepatocellular carcinoma (HCC). About 80% of infected patients with HCC display cirrhosis of the liver (Schafer, G.B. et al., Lancet 353:1253-1257 (1999)). There are an estimated 170 million infected individuals worldwide. Important modes of transmission involve frequent exposure to blood or blood products. Thus, illegal use of injectable drugs, high-risk sexual behavior, and use of plasma products or blood transfusions increase the risk of HCV infection.

Current therapies available for treating HCV infection include various types of interferon-α, including IFN-α₂ₐ and EFN-α_{2b} (INTRON^{®} A, Schering-Plough; ROFERON-A^{®}, Roche); pegylated form of interferon (PECT-DTrRON^{®}A, Schering-Plough); and combinations of interferons. Most patients, however, are unresponsive to these treatments, and among the responders, there is a high recurrence rate within 6-12 months after cessation of treatment (Liang et al., J. Med Virol. 40:69 (1993)). Sustained therapeutic responses are seen only after 1 to 2 years of extended interferon therapy. Ribavirin, a guanosine analog with broad-spectrum activity against many RNA and DNA viruses, has been shown in clinical trials to be effective against chronic HCV. infection when used in combination with interferon-α (see, *e.g.,* Poynard et al., Lancet 352:1426-1432 (1998); Reichard et al., Lancet 351:83-87 (1998)), and has been approved for therapeutic use (REBETRON, Schering-Plough). However, the response rate is still well below 50%. Therefore, additional compositions and methods for treating and/or preventing HCV infection are desirable, including enhancing systemic and targeted delivery of active anti-HCV compounds.

### 4. SUMMARY

A class of anti-viral compounds with a haloalkylamide moiety, various exemplary embodiments of which are more fully described in the detailed description section, are shown here to be metabolized by hydrolases, such as carboxylesterases, with subsequent loss of the compound's anti-viral properties. Identification of this metabolic pathway provides a basis for the development of various strategies to minimize metabolic transformation, and thereby enhance the therapeutic effectiveness of these anti-viral compounds. As will be appreciated by those skilled in the art, the descriptions and various embodiments in the present disclosure are also applicable to other compounds with a haloalkylamide moiety that are metabolized by hydrolases.

Accordingly, in various aspects, the present disclosure is directed to compositions and uses for protecting a compound with a haloalkylamide moiety from metabolism by a hydrolase. According to aspects of the present invention, there is provided:
a composition for use as a pharmaceutical comprising a compound that comprises a haloalkylamide moiety, and means for protecting the compound from metabolism by a hydrolase;
use of a compound that comprises a haloalkylamide moiety and a carboxylesterase inhibitor for the preparation of a medicament for treating a subject in need thereof;
use of a compound that comprises a haloalkylamide moiety for the preparation of a medicament that further comprises a carboxylesterase inhibitor for treating a patient in need thereof,
use of a compound that comprises a carboxylesterase inhibitor for the preparation of a medicament that further comprises a haloalkylamide moiety, for treating a patient in need thereof;
use of a carboxylesterase inhibitor for the preparation of a medicament for modulating the bioavailability of an anti-viral compound that comprises a haloalkylacetamide moiety in a subject;
use of a carboxylesterase inhibtor for the preparation of a medicament for modulating the bioavailability of an anti-viral compound that comprises a haloalkylamide moiety in a subject, comprising adjunctively administering to the subject the compound and a carboxylesterase inhibitor;
use of a composition of the present invention for the preparation of a medicament for inhibiting the metabolism of an anti-viral compound that comprises a haloalkylamide moiety in a subject, comprising administering to the subject.
In some embodiments, the composition and uses described herein are used to protect the anti-HCV compounds comprising the general structure of formula **(I),** or salts, hydrates, solvates or oxides thereof, to enhance their bioavailability and tissue delivery when used to treat or prevent HCV infections.

In some embodiments, the compositions comprise the haloalkylamide compound and an inhibitor of hydrolase activity, such as a carboxyesterase inhibitor. In some embodiments, useful inhibitors include, but are not limited to, a trifluoromethyl ketone compound, an organophosphate compound, an aminoacridine compound, a mixed ester compound, and carboxylesterase substrates. The inhibitors may be used individually or as a mixture to inhibit a specific carboxylesterase isozyme, to inhibit a plurality of isozymes, and/or inhibit carboxylesterases present in specific tissues responsible for mediating biotransformation.

In another aspect, the present disclosure provides for use of the hydrolase inhibitors in methods of treating or preventing HCV infection in a subject. In general, the uses comprise administering the anti-HCV compound and adjunctively administering a carboxylesterase inhibitor. The compounds may be administered together, such as in a pharmaceutical composition, administered separately, simultaneously, or sequentially, by the same route or by different routes. In some embodiments, the carboxylesterase inhibitor is administered prior to administration of the anti-HCV compound to pretreat the host, thereby inhibiting carboxylesterase activity before administration of the anti-HCV compound.

The adjunctive administration of the hydrolase inhibitor with the HCV compound provides a way of inhibiting the metabolism of the anti-viral compound by carboxylesterases and other hydrolases.

In some embodiments, the adjunctive administration of the carboxylesterase inhibitor with the anti-HCV compound provides a way of increasing the bioavailability of the anti-HCV compound in the treated host.

The present disclosure further provides various compositions comprising the haloalkylamide compound, such as the anti-viral compounds of structural formula (I), formulated to protect the compound from metabolism by hydrolases. In some embodiments, the compound is formulated as a sustained/delayed release composition in the form of microparticles and microcapsules, which bypasses the upper digestive tract and delivers the compound to the small intestine or large intestine, where carboxylesterase activities may be lower, or where direct uptake of the microcapsule may limit contact with carboxylesterases.
It is shown here that such formulations enhance the bioavailability of the anti-viral compounds.

In other embodiments, the protective compositions are compounds microencapsulated with an enteric polymer, where release of the compound is determined by the pH differences in the environs of the stomach, small intestine (or parts of the small intestine) and/or the large intestine. Specific embodiments of microencapsulated anti-viral compounds of structural formula (I) are based on enteric polymers ethyl cellulose or methacrylic acid/methyl methacrylate copolymers. In various embodiments, the compositions may be compounded as tablets or capsules for oral administration.

In a further aspect, the disclosure provides ways of using the microencapsulated anti-viral compounds for treating or preventing HCV infection in a subject.

### 5. BRIEF DESCRIPTION OF THE FIGURES

FIG. 1 illustrates the biotransformation of anti-HCV compounds of structure **II** (a substituted diphenyl heterocycle), and structure **III** (a substituted pyridyl heterocycle) to their corresponding aniline metabolites, structures **IV** and **V,** respectively.

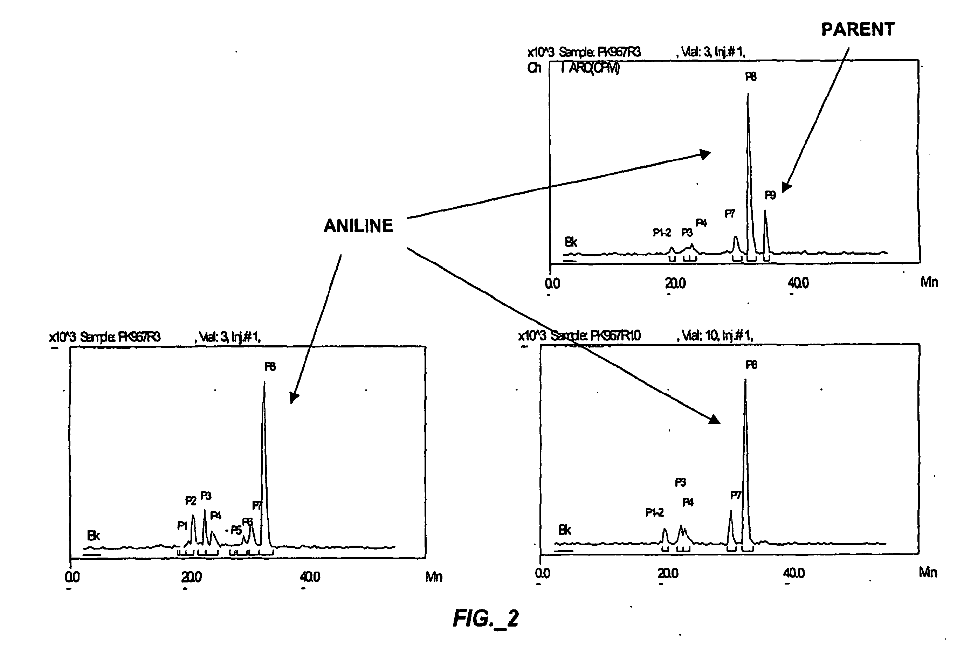

FIG. 2 shows the metabolite profile of ¹⁴C-labeled anti-HCV compound of **II.** Following administration of the compound to rats, plasma and liver were analyzed by HPLC for drug related metabolites. Aniline is the major product following intravenous administration of the substituted diphenyl heterocycle.

FIG. 3 shows the metabolic stability *in vitro* of **III.** The substituted pyridyl heterocyle was incubated with or without (control) human liver microsomes (male and female human liver microsomes: MF Human LM) in presence or absence of NADPH. Rapid disappearance of the parent compound occurs in microsomal samples in absence or presence of NADPH, an essential cofactor of cytochrome P-450 enzymes.

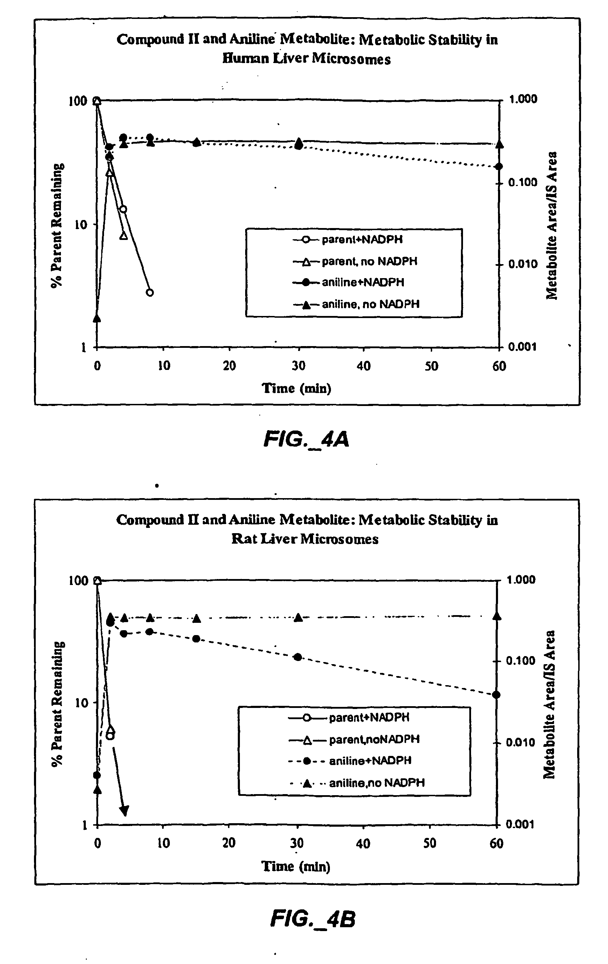

FIG. 4A and FIG. 4B show the stability of **II** when incubated in presence of microsomes prepared from human liver (FIG. 4A) or rat liver (FIG. 4B). Rapid disappearance of parent compound is seen along with concomitant appearance of long-lasting aniline metabolite. In presence of NADPH, the aniline product is slowly metabolized to hydroxylated products. Hydrolysis of **III** and its conversion to the corresponding aniline metabolite occurs in microsomes prepared from livers of human, monkey, dog, rabbit, rat, and mouse, with variation in rates of reaction per mg protein, depending on the species.

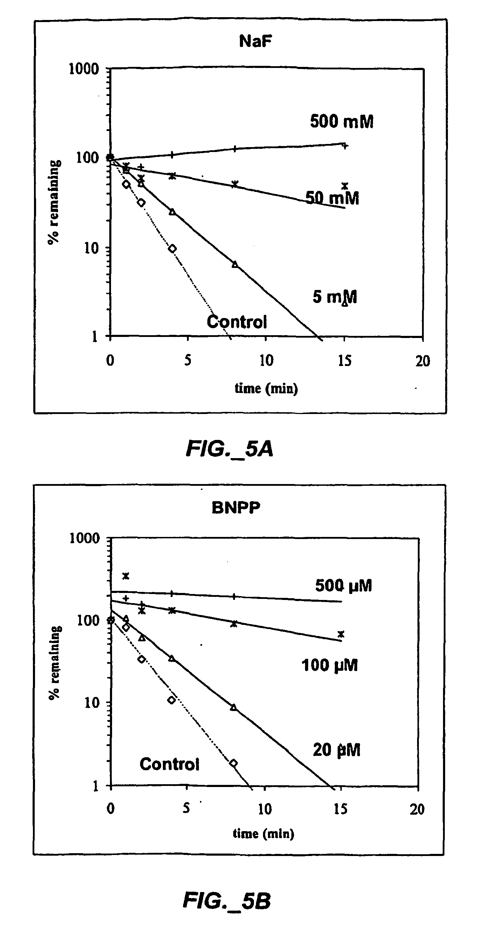

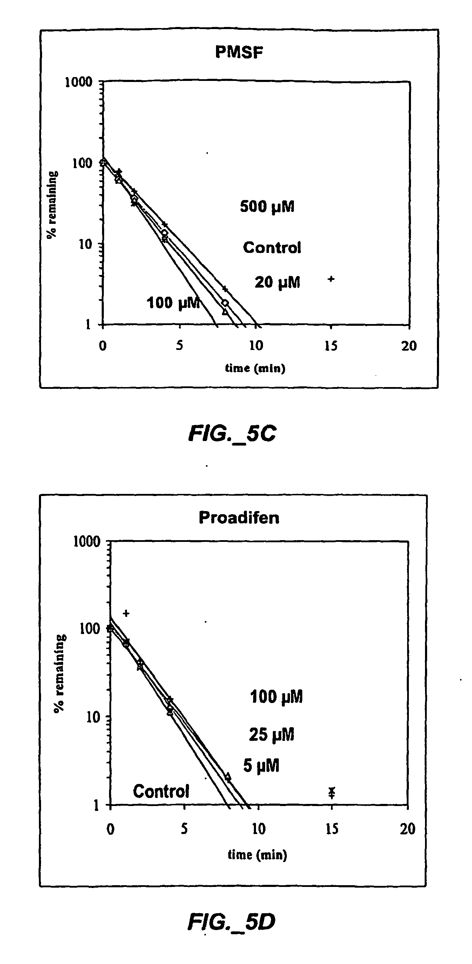

FIGS. 5A-5D show the effect of carboxylesterase and cytochrome P-450 inhibitors on the stability of **III** when incubated with human liver microsomes: FIG. 5A - NaF; FIG. 5B-BNPP; FIG. 5C - PMSF; and FIG. 5D - Proadifen. The concentration range of the inhibitors was as follows: NaF (5-500 mM), organophosphate BNPP (20-500 uM), PMSF (20-500 uM), and Proadifen (5-100 uM). Complete inhibition is observed at the highest concentrations of BNPP and NaF. PMSF shows weak inhibition. Proadifen, a cytochrome P-450 inhibitor, did not affect hydrolysis of the compound.

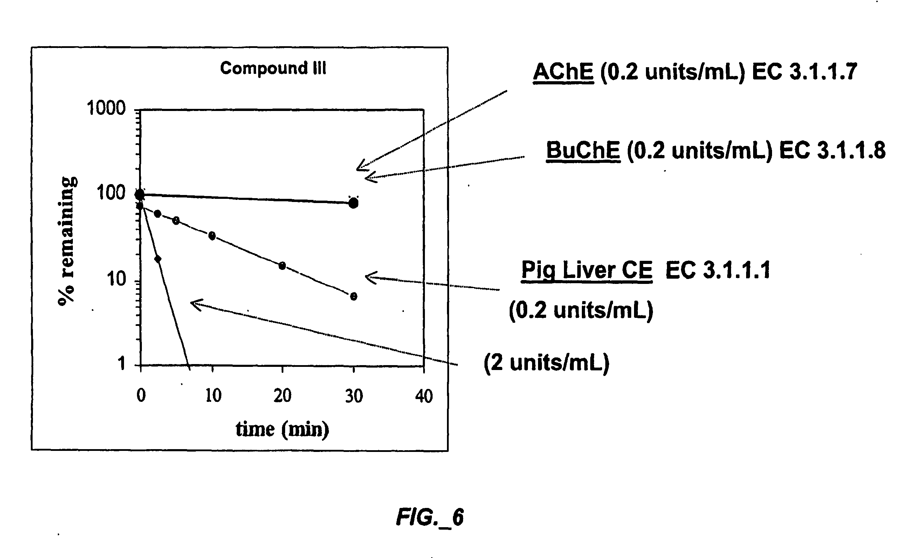

FIG. 6 shows the effect of various purified carboxyesterases on metabolism of **III.** Purified pig liver carboxylesterase (EC 3.1.1.1) efficiently hydrolyzed the compound in an enzyme concentration dependent manner. The compound is not an efficient substrate for acetylcholinesterase (AchE: EC 3.1.1.7) or butyrylcholinesterase (BuChE: EC 3.1.1.8).

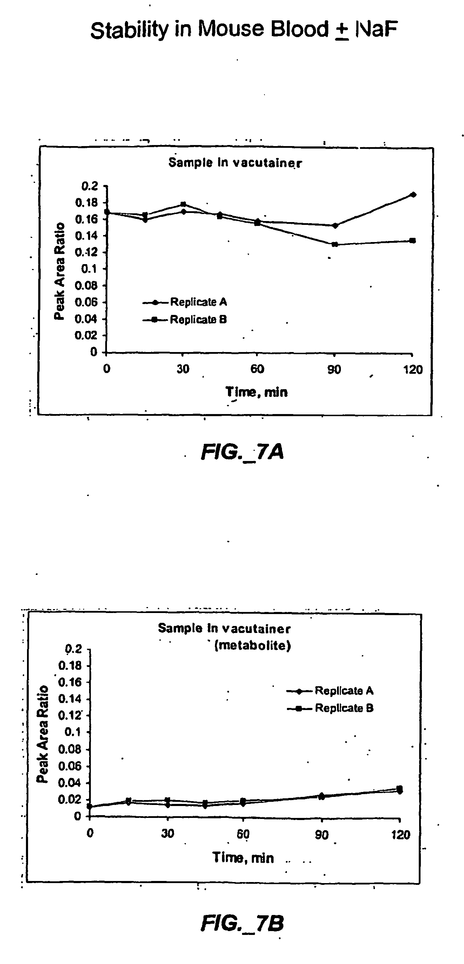

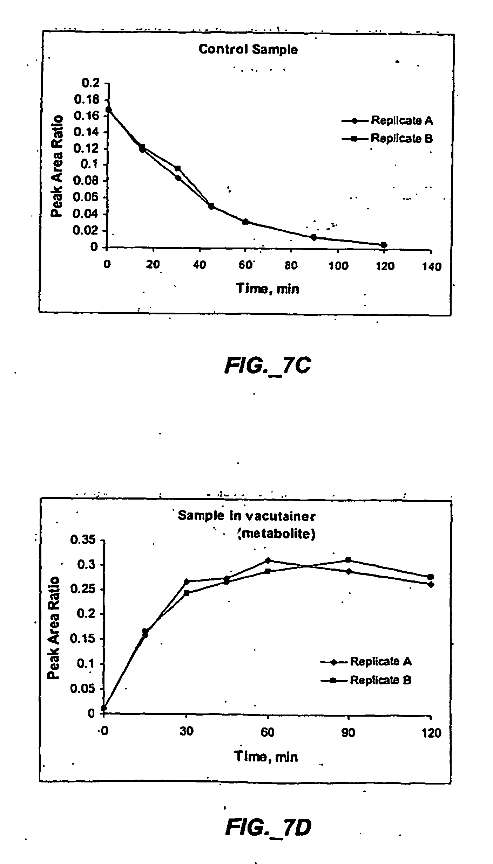

FIGS. 7A-7D show the stabilization of **III** in mouse blood in presence of 50 mM NaF. Inclusion of NaF prevented hydrolysis of the compound by plasma blood esterases, as seen by the levels of the compound remaining in the sample (FIG. 7A) and accumulation of metabolite (FIG 7B). In contrast, absence of NaF resulted in rapid decrease in the levels of compound in the sample (FIG. 7C) and rapid accumulation of the corresponding metabolite (FIG. 7D). Similar results are seen with organophosphate inhibitor BNPP. Thus, use of carboxylesterase inhibitors can preserve anti-HCV compounds present in biological specimens collected for analytical purposes.

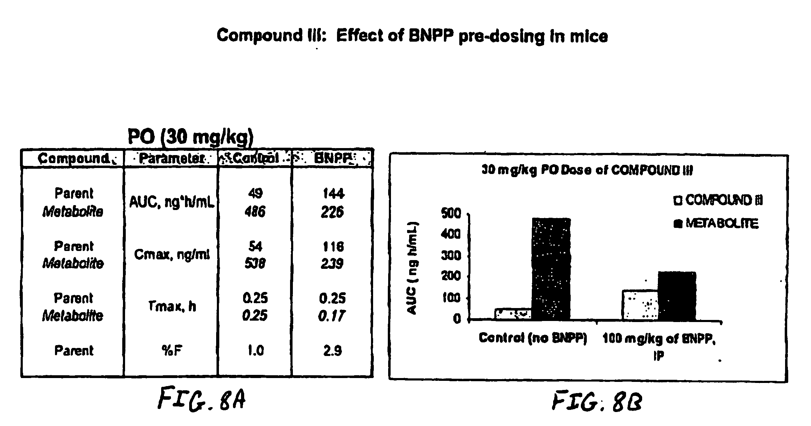

FIGS. 8A and 8B show enhancement of anti-HCV compound of structure **III** exposure in mouse when administered with carboxylesterase inhibitor BNPP. Following oral administration (PO), the animal has significantly reduced circulating metabolite and increased plasma exposure (AUC), Cₘₐₓ, and bioavailability (F) of the parent compound.

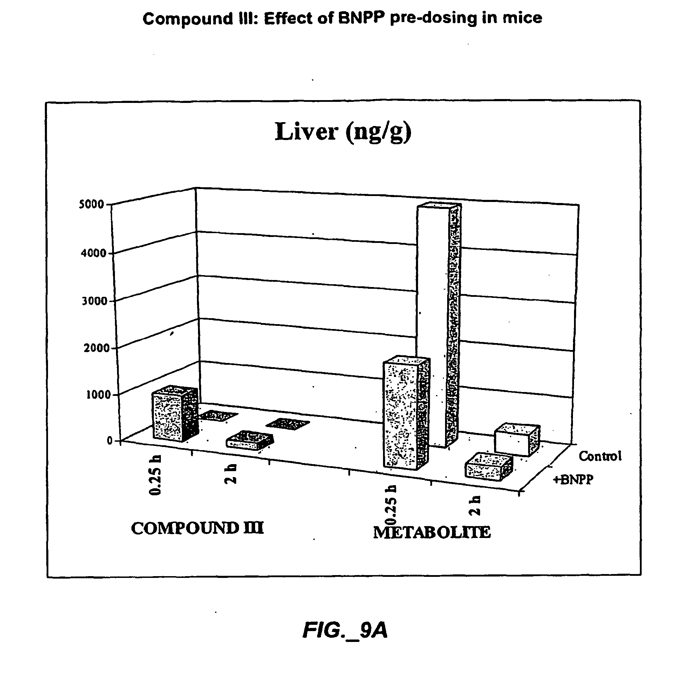

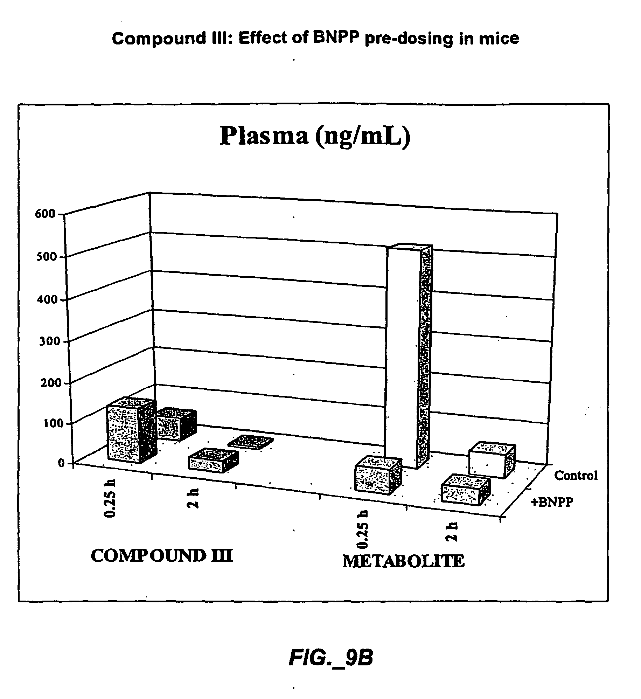

FIGS. 9A and 9B show the effect of pretreating mice with the carboxylesterase inhibitor BNPP on levels of parent compound and corresponding metabolite in the plasma and the liver. Animals treated with the carboxylesterase inhibitor have significantly increased levels of the parent compound in the liver, while insignificant levels are seen in untreated animals.

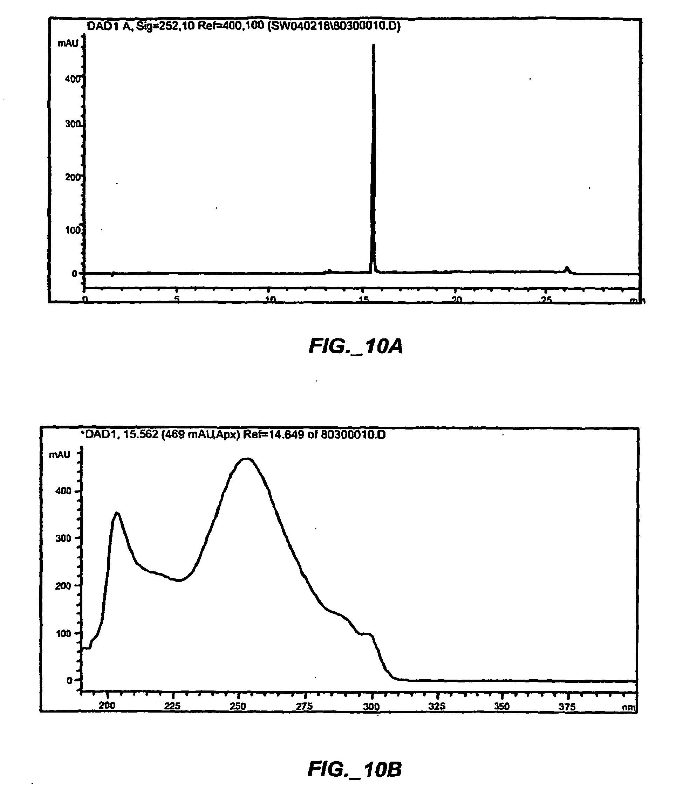

FIGS. 10A and 10B show a chromatogram and UV spectrum of a standard preparation of **III.**

FIG. 11A illustrates the appearance of microcapsule preparation Lot 1 (see Example 10) under polarized light microscope.

FIGS. 11B-11C show a chromatogram and UV spectrum of Lot 1.

FIG. 12A illustrates the appearance of microcapsule preparation Lot 2 under polarized light microscope.

FIG. 12B and 12C show a chromatogram and UV spectrum of Lot 2.

FIG. 13A and 13B show a chromatogram and UV spectrum of Lot 4.

FIG. 14A illustrates the appearance of microcapsule preparation Lot 6 under polarized light microscope.

FIG. 14B and 14C show a chromatogram and UV spectrum of Lot 6.

FIG. 15 shows the TGA profile of Lot 6.

FIG. 16A illustrates the appearance of microcapsule preparation Lot 7 under polarized light microscope.

FIG. 16B and 16C show a chromatogram and UV spectrum of Lot 7.

FIG. 17A illustrates the appearance of microcapsule preparation Lot 8 under polarized light microscope.

FIG. 17B and 17C show a chromatogram and UV spectrum of Lot 8.

FIG. 18A and 18B illustrates variations in the microcapsules formed at the top of the vessel (FIG. 18A) versus the bottom of the vessel (FIG. 18B) when the vessel is stirred at 250 rpm.

FIGS. 19A-19D are scanning electron micrographs of the outer surface (FIGS. 19A and 19B) or internal surface (FIGS. 19C and 19D) of the microcapsules from Lot 3.

FIG. 20 shows a differential scanning calorimetric profile of Eudragit S100 microcapsules of III over differing periods of time.

FIG. 22A illustrates a dissolution profile of Eudragit S100 microcapsules of III at pH 6.8 and 7.4.

FIG. 22B illustrates a dissolution profile of Eudragit L100 microcapsules of III at pH 6.8.

FIG. 23 illustrates concentration of III in cyno monkeys after oral administration of Eudragit S 100 microcapsules of III; Eudragit L100 microcapsules of III; enteric-coated polymorph of III; and TPGS solution of III.

### 6. DETAILED DESCRIPTION

### 6.1 Definitions

As used throughout the instant application, the following terms shall have the following meanings:

"Alkyl," by itself or as part of another substituent, refers to a saturated or unsaturated, branched, straight-chain or cyclic monovalent hydrocarbon radical derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-2-yl, prop-1-en-2-yl, prop-2-en-1-yl (allyl), cycloprop-1-en-1-yl; cycloprop-2-en-1-yl, prop-1-yn-1-yl, prop-2-yn-1-yl, *etc.*; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobute-1,3-dien-1-yl, but-1-yn-1-yl, but-1-yn-3-yl, but-3-yn-1-yl, *etc*.; and the like.

The term "alkyl" is specifically intended to include groups having any degree or level of saturation, *i.e.,* groups having exclusively single carbon-carbon bends, groups having one or more double carbon-carbon bonds, groups having one or more triple carbon-carbon bonds and groups having mixtures of single, double and triple carbon-carbon bonds. Where a specific level of saturation is intended, the expressions "alkanyl," "alkenyl," and "alkynyl" are used. Preferably, an alkyl group comprises from 1 to 15 atoms (C₁-C₁₅ alkyl), more preferably from 1 to10 carbon atoms (C₁-C₁₀ alkyl) and even more preferably from 1 to 6 carbon atoms (C₁-C₆ alkyl or lower alkyl).

"Alkanyl," by itself or as part of another substituent, refers to a saturated branched, straight-chain or cyclic alkyl radical derived by the removal of one hydrogen atom from a single carbon atom of a parent alkane. Typical alkanyl groups include, but are not limited to, methanyl; ethanyl; propanyls such as propan-1-yl; propan-2-yl (isopropyl), cyclopropan-1-yl, etc.; butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc.;* and the like.

"Alkenyl," by itself or as part of another substituent, refers to an unsaturated branched, straight-chain or cyclic alkyl radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, cycloprop-1-en-1-yl; cycloprop-2-en-1-yl ; butenyls such as but-l-en-1-yl, but-1-en-2-yl, 2-methyl-prop-1-en-1-yl, but-2-en-1-yl , but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, *etc.;* and the like.

"Alkynyl," by itself or as part of another substituent refers to an unsaturated branched, straight-chain or cyclic alkyl radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-ya-1-yl, etc.; butynyls such as but-1-yn-1-yl, but-1-yn-3-yl, but-3-yn-1-yl, etc.; and the like.

"Alkoxy," by itself or as part of another substituent, refers to a radical of the formula -OR, where R is an alkyl or cycloalkyl group as defined herein. Representative examples alkoxy groups include, but are not limited to, methoxy, ethoxy, propoxy, isopropoxy, butoxy, tert-butoxy, cyclopropyloxy, cyclopentyloxy, cyclohexyloxy and the like.

"Alkoxycarbonyl," by itself or as part of another substituent, refers to a radical of the formula -C(O)-alkoxy, where alkoxy is as defined herein.

"Alkylthio," by itself or as part of another substituent, refers to a radical of the formula -SR³¹, where R³¹ is an alkyl or cycloalkyl group as defined herein. Representative examples include, but are not limited to, methylthio, ethylthio, propylthio, isopropylthio, butylthio tert-butylthio, cyclopropylthio, cyclopentylthio, cyclohexylthio, and the like.

"Aryl," by itself or as part of another substituent, refers to a monovalent aromatic hydrocarbon group derived by the removal of one hydrogen atom from a single carbon atom of a parent aromatic ring system, as defined herein. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, *as*-indacene, *s*-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. Preferably, an aryl group comprises from 6 to 20 carbon atoms (C₆-C₂₀ aryl), more preferably from 6 to 15 carbon atoms (C₆-C₁₅ aryl) and even more preferably from 6 to 10 carbon atoms (C₆-C₁₀ aryl).

"Arylalkyl," by itself or as part of another substituent, refers to an acyclic alkyl group in which one of the hydrogen atoms bonded to a carbon atom, typically a terminal or *sp*³ carbon atom, is replaced with an aryl group as, as defined herein. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylalkenyl and/or arylalkynyl is used. Preferably, an arylalkyl group is (C₆-C₃₀) arylalkyl, *e.g.,* the alkanyl, alkenyl or alkynyl moiety of the arylalkyl group is (C₁-C₁₀) alkyl and the aryl moiety is (C₆-C₂₀) aryl, more preferably, an arylalkyl group is (C₆-C₂₀) arylalkyl, *e.g.*, the alkanyl, alkenyl or alkynyl moiety of the arylalkyl group is (C₁-C₈) alkyl and the aryl moiety is (C₆-C₁₂) aryl, and even more preferably, an arylalkyl group is (C₆-C₁₅) arylalkyl, *e.g.,* the alkanyl, alkenyl or alkynyl moiety of the arylalkyl group is (C₁-C₅) alkyl and the aryl moiety is (C₆-C₁₀) aryl.

"Aryloxy," by itself or as part of another substituent, refers to a radical of the formula -O-aryl, where aryl is as defined herein.

"Arylalkyloxy, by itself or as part of another substituent, refers to a radical of the formula -O-arylalkyl, where arylalkyl is as defined herein.

"Aryloxycarbonyl," by itself or as part of another substituent, refers to a radical of the formula -C(O)-O-aryl, where aryl is as defined herein.

"Carbamoyl," by itself or as part of another substituent, refers to a radical of the formula -C(O)NR'R", where R' and R" are each, independently of one another, selected from the group consisting of hydrogen, alkyl and cycloalkyl as defined herein, or alternatively, R" and R", taken together with the nitrogen atom to which they are bonded, form a cycloheteroalkyl ring as defined herein.

"Cycloalkyl," by itself or as part of another substituent, refers to a saturated or unsaturated cyclic alkyl radical, as defined herein. Where a specific level of saturation is intended, the nomenclature "cycloalkanyl" or "cycloalkenyl" is used. Typical cycloalkyl groups include, but are not limited to, groups derived from cyclopropane, cyclobutane, cyclopentane, cyclohexane, and the like. Preferably, the cycloalkyl group comprises from 3 to 10 ring atoms (C₃-C₁₀ cycloalkyl) and more preferably from 3 to 7 ring atoms (C₃-C₇ cycloalkyl).

"Cycloheteroalkyl," by itself or as part of another substituent, refers to a saturated or unsaturated cyclic alkyl radical in which one or more carbon atoms (and optionally any associated hydrogen atoms) are independently replaced with the same or different heteroatom. Typical heteroatoms to replace the carbon atom(s) include, but are not limited to, N, P, O, S, Si, *etc.* Where a specific level of saturation is intended, the nomenclature "cycloheteroalkanyl" or "cycloheteroalkenyl" is used. Typical cycloheteroalkyl groups include, but are not limited to, groups derived from epoxides, azirines, thiiranes, imidazolidine, morpholine, piperazine, piperidine, pyrazolidine, pyrrolidone, quinuclidine, and the like. Preferably, the cycloheteroalkyl group comprises from 3 to 10 ring atoms (3-10 membered cycloheteroalkyl) and more preferably from 3 to 7 ring atoms (3-7 membered cycloheteroalkyl).

"Dialkylamino" or "monoalkylamino," by themselves or as part of other substituents, refer to radicals of the formula -NRR and -NHR, respectively, where each R is independently selected from the group consisting of alkyl and cycloalkyl, as defined herein. Representative examples of dialkylamino groups include, but are not limited to, dimethylamino, methylethylamino, di-(1-methylethyl)amino, (cyclohexyl)(methyl)amino, (cyclohexyl)(ethyl)amino, (cyclohexyl)(propyl)amino and the like. Representative examples of monalkylamino groups include, but are not limited to, methylamino, ethylamino, propylamino, isopropylamino, cyclohexylamino, and the like.

"Halogen" or "halo," by themselves or as part of another substituent refer to a fluoro, chloro, bromo and/or iodo radical.

"Haloalkyl," by itself or as part of another substituent, refers to an alkyl group as defined herein in which one or more of the hydrogen atoms is replaced with a halo group. The term "haloalkyl" is specifically meant to include monohaloalkyls, dihaloalkyls, trihaloalkyls, etc. up to perhaloalkyls. The halo groups substituting a haloalkyl group can be the same, or they can be different. For example, the expression "(C₁-C₂) haloalkyl" includes 1-fluoromethyl,1-fluoro-2-chloriethyl difluoromethyl, trifluoromethyl, 1-fluoroethyl, 1,1-difluoroethyl, 1, 2-difluoroethyl, 1,1,1-trifluoroethyl, perfluoroethyl, etc.

"Heteroalkyl," "heteroalkanyl," "heteroalkenyl," "heteroalkynyl," "heteroalkyldiyl" and "heteroalkyleno," by themselves or as part of other substituents, refer to alkyl, alkanyl, alkenyl, alkynyl, alkyldiyl and alkyleno groups, respectively, in which one or more of the carbon atoms (and optionally any associated hydrogen atoms), are each, independently of one another, replaced with the same or different heteroatoms or heteroatomic groups. Typical heteroatoms or heteroatomic groups which can replace the carbon atoms include, but are not limited to, O S, N, Si, -NH-, -S(O)-, -S(O)₂-, -S(O)NH-, -S(O)₂NH- and the like and combinations thereof. The heteroatoms or heteroatomic groups may be placed at any interior position of the alkyl, alkenyl or alkynyl groups. Examples of such heteroalkyl, heteroalkanyl, heteroalkenyl and/or heteroalkynyl groups include -CH₂-CH₂-O-CH₃, -CH₂-CH₂-NH-CH₃, -CH₂-CH₂-N(CH₃)-CH₃, -CH₂-S-CH₂,-CH₃, -CH₂-CH₂-S(O)-CH₃, -CH₂-CH₂-S(O)₂-CH₃, -CH=CH-O-CH₃, -CH₂-CH=N-O-CH₃, and -CH₂-CH₂-O-C=CH. For heteroalkyldiyl and heteroalkyleno groups, the heteratom or heteratomic group can also occupy either or both chain termini. For such groups, no orientation of the group is implied.

"Heteroaryl," by itself or as part of another substituent, refers to a monovalent heteroaromatic radical derived by the removal of one hydrogen atom from a single atom of a parent heteroaromatic ring systems, as defined herein. Typical heteroaryl groups include, but are not limited to, groups derived from acridine, arsindole, carbazole, β-carboline, chromane, chromene, cinnoline, furan, imidazole, indazole, indole, indoline, indolizine, isobenzofuran, isochromene, isoindole, isoindoline, isoquinoline; isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. Preferably, the heteroaryl group comprises from 5 to 20 ring atoms (5-20 membered heteroaryl), more preferably from 5 to 10 ring atoms (5-10 membered heteroaryl). Preferred heteroaryl groups are those derived from thiophene, pyrrole, benzothiophene, benzofuran, indole, pyridine, quinoline, imidazole, oxazole and pyrazine.

"Parent Aromatic Ring System" refers to an unsaturated cyclic or polycyclic ring system having a conjugated π electron system. Specifically included within the definition of "parent aromatic ring system" are fused ring systems in which one or more of the rings are aromatic and one or more of the rings are saturated or unsaturated, such as, for example, fluorene, indane, indene, phenalene, etc. Typical parent aromatic ring systems include, but are not limited to, aceanthrylene, acenaphthylene, acephenanthrylene, anthracene, azulene, benzene, chrysene, coronene, fluoranthene, fluorene, hexacene, hexaphene, hexalene, *as*-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like.

"Parent Heteroaromatic Ring System" refers to a parent aromatic ring system in which one or more carbon atoms (and optionally any associated hydrogen atoms) are each independently replaced with the same or different heteroatom. Typical heteroatoms to replace the carbon atoms include, but are not limited to, N, P, O, S, Si, *etc.* Specifically included within the definition of "parent heteroaromatic ring system" are fused ring systems in which one or more of the rings are aromatic and one or more of the rings are saturated or unsaturated, such as, for example, arsindole, benzodioxan, benzofuran, chromane, chromene, indole, indoline, xanthene, etc. Typical parent heteroaromatic ring systems include, but are not limited to, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene and the like.

"Pharmaceutically acceptable salt" refers to a salt of a compound which is made with counterions understood in the art to be generally acceptable for pharmaceutical uses and which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like; or (2) salts formed when an acidic proton present in the parent compound is replaced by a metal ion, *e.g.*, an alkali metal ion, an alkaline earth ion, or an aluminum ion; or coordinates with an organic base such as ethanolamine, diethanolamine, triethanolamine, N-methylglucamine, morpholine, piperidine, dimethylamine, diethylamine and the like. Also included are salts of amino acids such as arginates and the like, and salts of organic acids like glucurmic or galactunoric acids and the like (see, *e.g.*, Berge et al., J. Pharm. Sci. 66:1-19 (1977).

"Prodrug" refers to a derivative of an active compound (drug) that undergoes a transformation under the conditions of use, such as within the body, to release an active drug. Prodrugs are frequently, but not necessarily, pharmacologically inactive until converted into the active drug. Prodrugs are typically obtained by masking a functional group in the drug believed to be in part required for activity with a progroup (defined below) to form a promoiety which undergoes a transformation, such as cleavage, under the specified conditions of use to release the functional group, and hence the active drug. The cleavage of the promoiety may proceed spontaneously, such as by way of a hydrolysis reaction, or it may be catalyzed or induced by another agent, such as by an enzyme, by light, by acid, or by a change of or exposure to a physical or environmental parameter, such as a change of temperature. The agent may be endogenous to the conditions of use, such as an enzyme present in the cells to which the prodrug is administered or the acidic conditions of the stomach, or it may be supplied exogenously. In a specific embodiment, the term prodrug includes hydro isomers of the compounds described herein. Such hydro isomers can be oxidized under physiological conditions to the corresponding aromatic ring system.

A wide variety of progroups, as well as the resultant promoieties, suitable for masking functional groups in active compounds to yield prodrugs are well-known in the art. For example, a hydroxyl functional group may be masked as a sulfonate, ester or carbonate promoiety, which may be hydrolyzed *in vitro* to provide the hydroxyl group. An amino functional group may be masked as an amide, imine, phosphinyl, phosphonyl, phosphoryl or sulfenyl promoiety, which may be hydrolyzed *in vivo* to provide the amino group. A carboxyl group may be masked as an ester (including silyl esters and thioesters), amide or hydrazide promoiety, which may be hydrolyzed *in vivo* to provide the carboxyl group. Other specific examples of suitable progroups and their respective promoieties will be apparent to those of skill in the art.

"Progroup" refers to a type of protecting group that, when used to mask a functional group within an active drug to form a promoiety, converts the drug into a prodrug. Progroups are typically attached to the functional group of the drug via bonds that are cleavable under specified conditions of use. Thus, a progroup is that portion of a promoiety that cleaves to release the functional group under the specified conditions of use. As a specific example, an amide promoiety of the formula -NH-C(O)CH₃ comprises the progroup -C(O)CH₃.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O⁻, =O, -OR^{b}, -SR^{b}, -S⁻, =S, -NR^{c}R^{c}, NR^{b}, =N-OR^{b}, trihalomethyl, -CF₃, -CN, -OCN, -SCN, -NO, -NO₂, =N₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{b} is independently hydrogen or R^{a}; and each R^{c} is independently R^{b} or alternatively, when each R^{c} taken together with the nitrogen atom to which they are bonded form a 5-, 6- or 7-membered cycloheteroalkyl which may optionally include from 1 to 4 of the same or different additional heteroatoms selected from the group consisting of O, N and S. As specific examples, -NR^{c}R^{c} is meant to include -NH₂, -NH-alkyl, N-pyrrolidinyl and N-morpholinyl.

Similarly, substituent groups useful for substituting unsaturated carbon atoms in the specified group or radical include, but are not limited to, -R^{a}, halo, -O⁻, -OR^{b}, -SR^{b}, -S⁻, -NR^{c}R^{c}, trihalomethyl, -CF₃, -CN, -OCN, -SCN, -NO, -NO₂, -N₃, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S⁻, NR^{c}R^{c}, trihalomethyl, -CF₃, -CN, -NO, NO₂, -S(O)₂R^{b}, -S(O)₂O⁻, -S(O)₂OR^{b}, -OS(O)₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)OR^{b}, -OC(S)OR^{b}, -NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The substituents used to substitute a specified group can be further substituted, typically with one or more of the same or different groups selected from the various groups specified above.

"Sulfamoyl," by itself or as part of another substituent, refers to a radical of the formula -S(O)₂NR'R", where R' and R" are each, independently of one another, selected from the group consisting of hydrogen, alkyl and cycloalkyl as defined herein, or alternatively, R' and R", taken together with the nitrogen atom to which they are bonded, form a 5-, 6- or 7-membered cycloheteroalkyl ring as defined herein, which may optionally include from 1 to 4 of the same or different additional heteroatoms selected from the group consisting of O, S and N.

"Carboxylesterase" refers to a subclass of hydrolases belonging to the superfamily of proteins characterized by an α,β hydrolase fold (Oakeshott, J.G. et al, Bioessay 21:1031-1042 (1999)). This structure is composed of alternating α-helix and β-sheets linked by amino acids sequences of varying length. Carboxylesterases are known to catalyze cleavage of carboxylic esters, amides, and thioester groups present on numerous chemical compounds, including drugs and xenobiotics.

"Isozyme" or "isoenzyme" refers to different, separable forms of an enzyme found in a species or the same organism and having similar or identical catalytic properties. Isozymes may be differentiated by variations in chemical, physical, or immunological properties, including, amino acid sequence, different modifications (*e.g.*, proteolytic processing), isoelectric point, electrophoretic mobility, kinetic parameters, reactivity with antibodies, or modes of regulation. The relative abundance of an isozyme may differ from one tissue to another and also change during the course of development. Further, different isozymes may result from association of polypeptide subunits that makeup the enzyme. Isozymes are often isoforms, which refer to any of a group of two or more different proteins that are produced by different genes and are specific to different tissues but have the same function and a similar sequence.

"Carboxylesterase inhibitor" refers to a compound or mixture of compounds which inhibit carboxylesterase activity. These include the art known definitions of competitive, non-competitive, uncompetitive, and mixed inhibitors. Inhibition may be reversible or irreversible. Within the scope of inhibitors are other carboxylesterase substrates capable of interfering with the cleavage of the substrates of interest.

"Pharmaceutically acceptable vehicle" refers to a diluent, excipient, or carrier with which a compound and/or inhibitor, or a composition thereof is administered.

"Pharmaceutically effective amount" refers to an amount sufficient to produce the desired physiological effect or amount capable of achieving the desired result, such as for treating a disorder or disease condition, including reducing or eliminating one or more symptoms of the disorder or disease or prevention of the disease or condition.

### 6.2 Metabolic Transformation of Compounds with a Haloalkylamide Moiety

Carboxyesterases are a subclass of hydrolases that belong to a superfamily of proteins characterized by an α,β hydrolase fold consisting of alternating α-helix and β-sheets linked by peptides of varying length (Oakeshott, J.G. et al, Bioessay 21:1031-1042 (1999)). Generally, carboxylesterases catalyze cleavage of carboxylic ester, amide, and thioester groups present on numerous chemical compounds. The enzymes are known to have wide specificity and act on substrates that include short and long acyl glycerols, long chain fatty-acyl-CoA esters and thioesters, aryl esters, lysophosphatidylcholine, acetic ester, acylcarnitine, aryacyl amides, and vitamin A esters. Carboxylesterases are also involved in biotransformation of a wide variety of drugs and xenobiotics containing ester, thioester, and amide functional groups. It is believed that one role for the biotransformation is to convert the apolar esters or amides to more soluble metabolites for removal from the body. Esterases and other hydrolases involved in drug metabolism are described in Testa, B. and Mayer, J. M., Hydrolysis in Drug and Prodrug Metabolism: Chemistry, Biochemistry, and Enzymology, Verlag Helvetica Chimica Acta, Zurich (2003), incorporated herein by reference in its entirety.

Carboxylesterases are expressed in numerous organisms, including bacteria, yeast, plants, insects, birds, and mammals. Carboxylesterases display low substrate specificity such that several carboxyesterases may be responsible for biotransformation of a single substrate, drug, or xenobiotic. Different carboxylesterase forms have been identified in mouse, rat, hamster, guinea pig, rabbit, dog, pig, cow, monkey, and humans, and categorized based on a number of biochemical criteria, such as substrate/inhibitor specificity, molecular weight, isoelectric point, cellular distribution, inducibility, etc. (Satoh, T. and Hosokawa, M., Ann. Rev. Pharmacol. Toxicol. 38:257-288 (1998)).

At the cellular level, carboxylesterases are present in the extracellular, microsomal, lysosomal, and cytosolic environments. The microsomal forms typically have an endoplasmic retention signal at the carboxy terminal region, which positions the enzyme on the luminal side of the endoplasmic recticulum. Carboxylesterases are also secreted into the extracellular fluid, such as blood. These secreted forms lack the endoplasmic recticulum retention signals found in the microsomally localized enzymes. Corresponding to the known cellular and extracellular location of these enzymes, many carboxylestease proteins have a signal peptide sequence for membrane insertion and membrane localization.

Nucleic acids encoding carboxylesterases have been isolated from rat, rabbit, mouse, hamsters, and human sources. Comparison of the deduced amino acid sequences indicates that the enzymes fall within four major groups, designated as CES 1, CES 2, CES 3, and CES 4 (Satoh, *supra).* CES 1 can be divided into three subfamilies and includes the identified isoforms of human carboxylesterases as well as major forms of dog, rabbit, rat and mouse carboxylesterases. CES 2 includes rabbit form 2 and hamster carboxylesterase AT-57. CES 3 includes the mouse ES-male enzyme and the HU3 carboxylesterase. CES 4 includes the 46.5 kDa carboxylesterase isozymes identified in humans, monkey liver, and mouse liver (Satoh, *supra*)*.* This latter class of enzymes has lowest similarity to hCE-1 and is believed to be responsible for metabolic activation of arylamine and heterocyclic amine carcinogens.

Three major types of human carboxyesterases, HU1, HU2, HU3 have been characterized biochemically and molecularly. Human carboxylesterase-1 (hCE-1, CES-1, HU1) is a 180 kD trimeric protein, displaying selectivity for the methyl ester of cocaine and heroin ester. Human carboxyesterase-2 (hCE-2) is a 60 kD monomeric enzyme, displaying selectivity against benzoyl ester of cocaine, 6- and 3- acetyl groups of heroin, acetylsalicylic acid, and oxybutynin (Pindel, E.V. et al., J. Biol. Chem. 272(23):14769-75 (1997)). hCE-2 is also specific for 4-methylumbelliferyl acetate, a compound initially described as a nonspecific esterase substrate (Humerickhouse, R. et al., Cancer Res. 60(5):1189-1192 (2000)). hCE-3 is related to the human brain carboxyesterases (HBR3) (Mori, M. et al., FEBS Lett. 458(1):17-22 (1999)) and is found in the brain and adrenal gland.

In addition to the major forms described above, carboxylesterase expressed as a mRNA of 3.4-3.6 kb has been identified from intestine. This form displays only minor expression in the liver (Schwer, H. et al., Biochem Biophys Res Commun. 233(1):117-120 (1997)). Within the intestine itself, highest expression of the RNA is found in the small intestine with reduced levels in colon and rectum. There is differential expression of this carboxylesterase within the small intestine itself, with the highest expression seen in the jejunum compared to duodenum and ileum.

The various human carboxylesterase isozymes exhibit differences in selectivity in substrate recognition and cleavage. For example, both hCE-1 and hCE-2 bind opiod ester meperidine (*i.e.,* Demerol), but only hCE-1 efficiently cleaves the drug to the corresponding acid and ethanol (Zhang, J. et al., J. Pharm. Exp. Ther. 290(1):314-318 (1999)). Marked differences in substrate selectivity are also evidenced by activities against paranitrophenylacetate and paranitrophenyl butyrate. hCE-1 does not display a preference for either substrate; hCE-2 preferentially hydrolyzes paranitrophenylacetate; and hCE-3 preferentially hydrolyzes paranitrophenylbutyrate (Xie, M. et al., Drug Metab. Disp. 30(5):541-547 (2002)). Another example of differential specificity is the activation of chemotherapeutic drug irinotecan. hCE-2 acts on the amide prodrug, but hCE-1 is ineffective in converting the drug to its active form. These differences in enzyme specificity are also reflected in sensitivity to various carboxylesterase inhibitors. For instance, hCE-3 has higher sensitivity to phenylmethylsulfonyl fluoride (PMSF) while HCE-1 and HCE-2 are less sensitive. In comparison, HCE-1 and HCE-3 are sensitive to inhibition by paraoxon while HCE-2 is resistant (Xie, et al., *supra*).

Mammalian carboxylesterases show the highest expression in the liver, but are also present in other tissues, including small intestine, large intestine, testis, kidney, heart, skin, muscle, lung, stomach, brain, and plasma. Studies of hCE-1 expression show that it is present as a single mRNAs species, of which the expression levels are liver >> heart > stomach > testis = kidney = spleen > colon > other tissues. hCE-2 is expressed as three mRNA species. A 2 kb mRNA is expressed at various levels in the order of liver > colon > small intestine > heart, while a 3 kb message is found in liver > small intestine > colon > heart. A 4.2 kb message is found exclusively in brain, testis, and kidney. Studies with antibodies specific to hCE-1 show protein levels corresponding to the mRNA levels, with highest expression seen in the liver, followed by heart, prostate, lung, and ileum (Xie et al, *supra*)*.* Weak protein expression is detected in testis, spleen, and brain. Antibodies specific to hCE-2 show that the enzyme is highly expressed in liver, kidney, and ileum, while lower levels of expression are seen in the duodenum and adrenal gland. hCE-2 protein levels are not detected or are present at low levels in all other tissues. hCE-3 protein is found at highest levels in the adrenal gland and in lower amounts in the brain (Zhang, W. et al., Appl. lmmunohistochem. Mol. Morphol. 10(4):374-80 (2002); Xie et al., *supra*). It is believed that hCE-1 is primarily responsible for clearance through the kidney while hCE-2 is major pathway responsible for clearance through the small intestine and colon. Certain tissues, such as the kidney, adrenal and ileum may express all three isozymes. Liver, which contains the highest level of carboxylesterase activity, fails to express hCE-3.

It has now been demonstrated that hydrolases, specifically carboxylesterases, act on compounds that comprise a haloalkylamide moiety. An exemplary embodiment of a haloalkylamide moiety is a *gem*-dichloroacetamide moiety. This metabolic transformation is exemplified for a class of anti-viral compounds having a five membered ring with substituted diphenyl or pyridyl groups, where one of the substituents is a *gem*-dihaloacetamide group. The hydrolases appear to convert the anti-viral compounds to the corresponding aniline metabolite by removal of the dihaloacetamide group. Because the metabolic product is biologically inactive, the presence of hydrolase activities limits adsorption, bioavailability, and tissue delivery of these anti-viral compounds in a treated host. It is further demonstrated herein that that absorption, and tissue delivery of these anti-HCV compounds may be enhanced by protecting the antiviral compounds from metabolism by the hydrolases. This may be achieved by various compositions and methods, such as use of hydrolase enzyme inhibitors and/or formulations that protect the compound from metabolism by the hydrolases.

The exemplary class of anti-HCV compounds which serve as substrates for the hydrolases share certain structural similarities and are generally characterized by three main features: (i) a substituted 6-membered aromatic **"A"** ring; (ii) a substituted or unsubstituted 5-membered saturated, unsaturated or aromatic **"B"** ring; and (iii) a substituted 6-membered aromatic **"C"** ring. These anti-viral compounds have the general structure of formula (I):

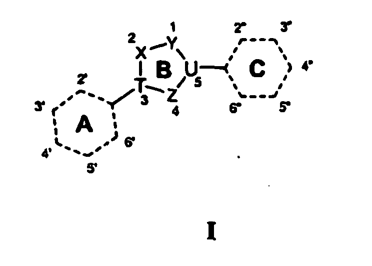

The depiction of the **"A", "B"** and **"C"** rings in this format is merely for schematic purposes only and is not meant to exclude the use of heteroatoms within any of these rings. Indeed, in many embodiments one or both of the **"A"** and **"C"** rings includes a nitrogen heteroatom and the **"B"** ring includes from one to four of the same or different heteroatoms selected from N (or NH), O and S.

In many of these compounds, the **"C"** ring is substituted at the *meta* position (3" or 5") with a substituent of the formula -NR¹¹C(O)R¹², where R¹¹ is hydrogen, methyl or other alkyl and R¹² is a substituted alkyl, haloalkyl, dihalomethyl, dichloromethyl., cycloheteroalkyl or substituted cycloheteroalkyl. In some embodiments, R¹¹ is a haloalkyl or a dichloromethyl group. In a specific embodiment, R¹¹ is hydrogen and R¹² is dichloromethyl. It is the haloalkylamide group which is the moiety acted upon by carboxylesterases. The **"C"** ring may also be optionally substituted at one or more of the 2", 4", 5" and/or 6" positions with the same or different halo groups.

The **"A"** ring includes at least one substituent positioned *ortho* (2" or 6" position) and may optionally include one or more of the same or different substituents positioned at the other ring positions. The nature of the substituents can vary broadly and include halo, fluoro, chloro, alkyl, alkylthio, alkoxy, alkoxycarbonyl, arylalkyloxycarbonyl, aryloxycarbonyl, cycloheteroalkyl, carbamoyl, haloalkyl, dialkylamino or sulfamoyl groups and substituted versions thereof. In some embodiments, the **"A"** ring bears the same or different substituents at the 2" and 6" positions and is unsubstituted at the 3", 4" and 5" positions.

Exemplary embodiments of the HCV inhibitory compounds include compounds in which both of the **"A"** and **"C"** rings are substituted phenyl groups, compounds in which one or both of the **"A"** and **"C"** rings are pyridyl groups, for example pyrid-2-yl groups, and compounds in which the **"B"** ring is an aromatic ring comprising one, two or three of the same or different heteroatoms or heteroatomic groups selected from N, NH, O and S.

The identity of the **"B"** ring can vary broadly. In some embodiments, the **"B"** ring is a heterocyclic ring selected from isoxazolyl, pyrazolyl, oxadiazolyl, oxazolyl, thiazolyl, imidazolyl, triazolyl, thiadiazolyl and hydro isomers. Suitable hydro isomers include, but are not limited to, dihydro and tetrahydro isomers of the stated rings. Specific examples of such hydro isomers include, for example, 2-isoxazolinyls, 3-isoxazolinyls, 4-isoxazolinyls, isoxazolidinyls, 1,2-pyrazolinyls, 1,2-pyrazolidinyls, (3H)-dihydro-1,2,4-oxadiazolyls, (5H)-dihydro-1,2,4-oxadiazolyls, oxazolinyls, oxazolidinyls, (3H)-dihydrothiazolyls, (5H)-dihydrothiazolyls, thiazolidinyls (tetrahydrothiazolyls), (3H)-dihydrotriazolyls, (5H)-dihydrotriazolyls, triazolidinyls (tetrahydmtriazolyls), dihydro-oxadiazolyls, tetrahydro-oxadiazolyls, (3H)-dihydro-1,2,4-thiadiazolyis, (5H)-dihydm-1,2,4-thiadiazolyls, 1,2,4-thiadiazolidinyls (tetrahydrothiadiazolyls), (3H)-dihydroimidazolyls, (5H)-dihydroimidazolyls and tetrahydroimidazolyls.

These exemplary embodiments of the compounds of structural formula **(I)** above, including various prodrugs, solvates, oxides and salts thereof, as well as specific species of these compounds and methods for their synthesis, are described in the following copending applications: U.S. Patent Publication No. 20040127497 (Serial No. 10/646,348); U.S. Patent Publication No. 2003/0165561 (Serial No. 10/286,017); U.S. application Serial No. 60/467,650 filed May 2, 2003; U.S. application Serial No. 60/467,811, filed May 2, 2003; WO 03/040112 (PCT/US02/35131); U.S. application Serial No. 60/467,650, filed May 2, 2003; PCT/C1S2004/013452, filed April 30, 2004; U.S. application Serial No. 10/838,133, filed May 3, 2004; U.S. Patent Publication No. 2004/0142985 (Serial No. 10/440,349); and WO 2004/018463 (PCT/US03/026478). Exemplary embodiments of the anti-HCV compounds are specifically disclosed in PCT/US2004/015665 (see page 26). The disclosures of each of these applications are incorporated herein by reference in their entireties. All of the exemplary embodiments in this application fall within the class of compounds described in the references above.

The principles and features of the compositions and methods of use will be described for exemplary haloalkylamide substituted diphenyl heterocycle of structural formula **(II)** and exemplary haloalkylamide substituted pyridyl heterocycle of structural formula **(III)**

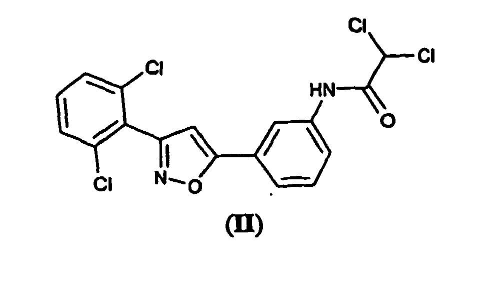

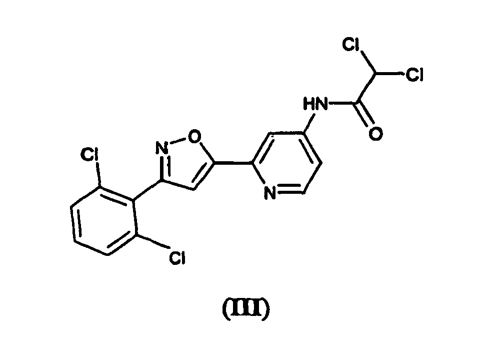

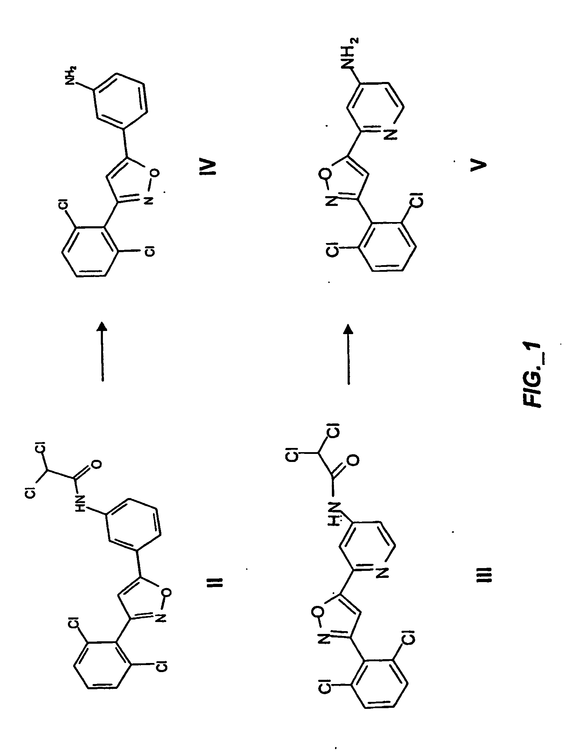

Compounds of structural formula **(II)** and **(III)** are converted to the corresponding aniline metabolites (see FIG. 1, structural formula **(IV)** and **(V)**) by hydrolysis of the dichloroacetamide group. Protecting the dichloroacetamide group of these specific compounds from metabolism by carboxylesterases appears to enhance bioavailabilty and tissue delivery of the compounds. This finding provides a basis for enhancing therapeutic effectiveness of anti-viral compounds with a haloalkylamide group by protecting the compounds from inactivation by hydrolase enzymes.

Accordingly, the disclosure provides compositions and methods that protect a compound comprising a haloalkylamide moiety from being metabolized by a hydrolase, such as a carboxylesterase. An exemplary haloalkylamide moiety to be protected is a *gem-*dichlomacetamide moiety.

In some embodiments, the compounds comprising the haloalklamide moiety are the anti-HCV compounds of structural formula **(I),** wherein:
the **"A"** ring comprises a substituted phenyl or a substituted pyridyl;
the **"B"** ring comprises a saturated, unsaturated or aromatic 5-membered ring that optionally includes a heteroatom at one or more of positions X, Y, and Z, each heteroatom being independently selected from NH, N, O and S, with the proviso that X and Y are not both O;
the **"C"** ring comprises a phenyl or pyridyl that is substituted at the 3" or 5" position with a haloalkylamide moiety of the formula -NR¹¹C(O)R¹², where R¹¹ is hydrogen or alkyl and R¹² is a haloalkyl, a dihalomethyl, or a dichloromethyl, and which may optionally include one or more of the same or different additional unillustrated substituents described above.

In some embodiments of the anti-viral compounds, the **"A"** and **"C"** rings are phenyl, or one or both of the **"A"** and **"C"** rings are a pyridyl. Antiviral compounds of particular interest are those in which R¹¹ is hydrogen and R¹² is a dichloromethyl. Exemplary anti-viral compounds for use in the compositions are the compounds **II** and **III.**

As will be appreciated by the skilled artisan, while the various embodiments are drawn to the anti-HCV compounds with the haloalkylamide moiety, other compounds that include this haloalkylamide moiety, for example a dichloroacetamide, could be protected from inactivation using the compositions and methods of the present disclosure.

Compositions and methods for protecting the compounds from metabolic transformation by hydrolases, such as by carboxylesterases, may rely on a number of different approaches. In some embodiments, the compositions and methods may be based on inhibiting the activity of the enzymes responsible for metabolism of the haloalkylamide containing compounds. Thus, the disclosure provides compositions of anti-HCV compounds and carboxylesterase inhibitors and methods of using the carboxylesterase inhibitors to protect the compounds from inactivation.

In other embodiments, the compositions and methods for protecting the haloalkylamide compounds from hydrolysis comprise formulating the compounds to avoid or reduce contact with hydrolases. For orally administered compounds, this may be done by formulating the compound for release in the small intestine or large intestine, where carboxylesterase activities may be lower as compared to the stomach. In addition, the direct uptake by the small intestine of microparticles containing the compound may bypass regions of carboxylesterase activities. Thus, in certain embodiments, as further described below, the disclosure is directed to compositions of compounds formulated as orally administered sustained/delayed release microparticles, with exemplary embodiments directed to compounds microencapsulated in enteric polymers.

### 6.3 Hydrolase Inhibitors

For embodiments where enzyme inhibitors are used to protect the haloalkylamide group from being metabolized, a variety of carboxylesterase inhibitors may be used with the compounds, either independently or in combination, such as in a composition. Further, it is to be understood that although the following descriptions are given for carboxylesterase enzymes and carboxylesterase inhibitors, other hydrolases, including other esterases, that act to metabolize the compounds may be similarly targeted using enzyme inhibitors known in the art. As such, useful inhibitors are "anti-HCV compound hydrolase inhibitors", which refer to compounds that inhibit hydrolysis of the haloalkylamide moiety on the compounds of structural formula (I).

In some embodiments, the carboxylesterase inhibitor is NaF, a general inhibitor of esterases with demonstrated inhibitory activity against the carboxylesterases that act on the anti-HCV compounds. NaF fluoride is believed to act in a competitive manner with the active site of esterases (Haugen, D.A. and Sutter, J.W., J. Biol. Chem. 249(9):2723-2731 (1974); Dulac, R.W. and Yang, T.J., Exp. Hematol. 19:59-62 (1991)). Although having limited *in vivo* applications because of toxicity, NaF is useful in inhibiting enzyme activity *in vitro,* such as in blood samples, cell culture, and cell extracts.

In other embodiments, the carboxylesterase inhibitors are trifluoromethyl ketones (TFK) and derivatives thereof (Wheelock, C.E. et al., J. Med Chem. 45:5576-5593 (2002); Zhang, J-G. and Fariss, M.W., Biochem. Pharm. 63:751-754 (2002); publications incorporated herein by reference). Without being bound by theory, it is thought that these inhibitors act as transition state analogues by forming tetrahedral intermediates via nucleophilic attack on the carbonyl carbon of the TFK by an enzyme catalytic residue. The tetrahedral complex is stable to cleavage and remains tightly bound to the enzyme. Various derivatives of TFKs are known in the art. Exemplary TFK inhibitors with modifications at the β atom to the carbonyl are described in Hammock, B.D. et al., Pestic. Biochem. Physiol. 17:76-88 (1982), and Ashour, M. and Hammock, B.D., Biochem. Pharmac. 36:1869-1879 (1987); modifications involving unsaturation at the α atom are described in Linderman, R.J. et al., Pestic. Biochem. Physio/. 35:291-299 (1989); alkyl substitutions at the α and γ atoms are described in Linderman, R.J. et al., Pestic. Biochem. Physiol. 29:266-277 (1987); and ether derivatives are described in Wheelock, *supra.* Inhibitors similar to TFKs include, but are not limited to, chlorodifluoracetaldehyde, and thioesters such as a alkyl thioacetothioates (Huang, T.L. et al., Pharm. Res. 13(10):1495-1500 (1996), hereby incorporated by reference).

In further embodiments, the carboxylesterase inhibitors may be organophosphates and their derivatives. Similar to trifluoromethyl ketone based inhibitors, organophosphorous inhibitors are believed to act as transition state analogues in which a tetrahedral intermediate is formed by a nucleophilic attack on the phosphorous atom by the enzyme catalytic residue. The enzyme-complexed phosphate ester hydrolyzes extremely slowly, thereby inhibiting further catalytic activity. Exemplary organophosphorous esterase inhibitors include, but are not limited to, phenylmethylsulfonylfluoride (PMSF); diisopropylphosphorofluoridate (DSF); sarin; parathion; tetrachorovinphos (TCVP); tri-orthocresylphosphate; phenyl phenylphosphonothioates (*e.g.,* leptophos); bis-p-nitrophenylphosphate (BNPP); phosphorine compounds, such as Bomin-2 (2-alkoxy-2-oxo-3-H-1,4,2-benzodioxaphosphorine) and Bomin-3 (2-alkoxy-2-oxo-3-H-1,4,2-benzodioxaphosphorine); and diethyl p-nitrophenylphosphate. For use in pharmaceutical compositions, organophosphorous inhibitors that do not have significant toxic inhibitory activities against brain cholinesterases, such as acetylcholinesterases, are selected.

In some embodiments, the carboxyesterase inhibitors are based on heterocylic compound acridine (Bencharit, S. et al., Chem. Biol. 10:341-349 (2003); hereby incorporated by reference). Structural analysis of co-crystals of hCE-1 enzyme and carboxylesterase inhibitor 9-aminoacridine (tacrine) shows that the inhibitor binds in various orientations to a promiscuous substrate-binding site (Bencharit, *supra).* A large binding pocket at the catalytic site is able to accommodate a spectrum of substrates and interact in multiple orientations, which enhances binding of different substrates to the enzyme. Exemplary acridine type inhibitors useful in the compositions and methods include aminoacridines, such as 9-aminoacridine, and derivatives selective for human carboxylesterases, for example 6,9-diamino-2-ethoxyacridine and 9-amino-6-chloro-2-methoxyacridine, both of which are selective for hCE-1 (Bencharit, *supra*).

Other types of carboxylesterase inhibitors useful in the compositions and methods include, but are not limited to, tetraethylthioperoxydicarbonic diamide (disulfiram) and nondihydroguaiaretic acid (NGDA) and its derivatives, such as heminordihydroguaiaretic acid (HNDGA) and norisoguaiacin (Schegg, K.M. and Welch, W., Biochem. Biophys. Acta 788:167-180 (1984)). Disulfiram, known as an inhibitor of alcohol dehydrogenase, appears to inhibit both plasma and microsomal esterases. NGDA also has broad specificity for carboxylesterases, possibly because of the flexibility of the molecule and its amphipathic character, which in combination allows for interaction with the binding sites of numerous types of carboxylesterases.

Other embodiments of carboxylesterase inhibitors are esters and ester mixtures, which appear to compete as substrates for carboxylesterases, for example in the intestine when administered orally as an adjunct to oral delivery of a drug acted on by intestinal carboxylesterases (van Gelder, J. et al., Pharm. Res. 16(7):1035-1040 (1999); van Gelder, J. et al., Drug Metab. Disp. 30(8):924-930 (2002); Testa and Mayer, *supra*; publications incorporated herein by reference). By using an ester or a heterogeneous collection of ester substrates, it is possible to inhibit carboxylesterase activity sufficiently to reduce biotransformation. Natural sources of esters and mixed esters include, but are not limited to, those derived from fruits and vegetables, such as strawberries, apricot, and banana. Synthetically produced mixed or discrete esters, similar to the natural esters, may serve as efficient substitutes (van Gelder, J. et al, Drug Metabolism Disp. 30(8):924-930 (2002); incorporated herein by reference).

As will be appreciated by those skilled in the art, other carboxylesterase substrates or substrate mimics that interfere with the cleavage of the anti-HCV compounds described herein may be used as the inhibitors. These include compounds with carboxyl, ester, and amide groups. Such exemplary compounds include, by way of example and not limitation, physostigmine (eserine); sulfonamides and organic amides; and FR182877, a selective inhibitor of carboxylesterase-1. Sulfonamides are derivatives of para-aminobenzenesulfonamide and include, but are not limited to, sulfadiazine, sufamethoxazole, sulfaoxazole, sulfaacetamide, and para-aminobenzoic acid. Any other drugs, such as ester and ester prodrugs, metabolized by carboxylesterases may also be used as inhibitors of carboxylesterases, as described in Testa, *supra,* and Satoh, T. et al., Drug Metab. Dispos. 30(5):488-493 (2002), hereby incorporated by reference.

In the compositions and methods described herein, broad-spectrum carboxylesterase inhibitors may be used with the anti-HCV compounds to inhibit multiple carboxylesterases. The use of a relatively general inhibitor allows use of a single inhibitor to inhibit carboxylesterases with overlapping activity directed against the anti-HCV compounds. Suitable inhibitors of this type include NaF, 9-aminoacridine, and diisopropylfluoride compounds.

In other embodiments, the inhibitors used are relatively specific to one type or class of carboxylesterases. Inhibitors with high degree of specificity will be useful for inhibiting a specific carboxylesterase isozyme. It is known that carboxylesterases display selectivity in substrate recognition despite their broad specificity, as seen by the varying Kₘ for different esterase substrates and differing sensitivities to esterase inhibitors (Satoh, *supra).* For the human carboxyesterases, hCE-1 is relatively more sensitive to inhibition by lactone compound FR182877 while hCE2 is relatively more sensitive to inhibitor by organophosphorous compound paraoxon. As the anti-HCV compounds described herein may be preferentially metabolized by a particular carboxylesterase isozyme, the compositions and methods provide for use of inhibitors selective for a carboxylesterase isozyme, including a human carboxyesterase isozyme. In some embodiments, these include inhibitors specific for HU1, HU2, or HU3.

Given that biotransformation of the anti-HCV compounds occurs through action of carboxylesterase activities in different tissues, carboxylesterase inhibitors may be used to inhibit enzyme activities present in specific tissues, including, but not limited to, liver, small intestine, large intestine, and blood. This may be accomplished through the selection of inhibitors relatively specific for carboxylesterases present in the specified tissue (see Table I), or through administration of the inhibitors in a manner (*e.g.,* intravenous, oral, rectal, etc.) sufficient to deliver the inhibitors preferentially to the target tissue (Satoh, T., et al., Drug Metab. Dispos. 30(5):488-493 (2002)).

**TABLE I^{a}**

| Compound **III:** Effect of carboxylesterase inhibitor BNPP on hydrolysis in human small intestinal and colon microsomes | | | | |
|---|---|---|---|---|
| Inhibitor | Concentration µM | Activity pmol/min/mg | Activity % | Inhibition % |
| BNPP | Human Intestine (pooled 2 subjects) | | | |
| | 0 | 1516 | 100 | 0 |
| | 50 | 432 | 28 | 72 |
| | 500 | 39 | 2.6 | 97 |
| BNPP | Human Colon, Subject 1 | | | |
| | 0 | 257 | 100 | 0 |
| | 50 | 192 | 75 | 25 |
| BNPP | Human Colon, Subject 2 | | | |
| | 0 | 192 | 100 | 0 |
| | 50 | 232 | 121 | 0 |

| | | | | |
|---|---|---|---|---|
| *^{a}*Microsomal protein 0.48 mg/mL; substrate concentration: 1.8 µM; activity was monitored by substrate disappearance. Initial rates were determined by linear regression of six independent incubations for 0, 1, 2.5, 5, 10, 20 min at 37°C following 10 min preincubation with inhibitor or vehicle. | | | | |

As further described in detail below, the tissue to be targeted will determined by the mode of administration of the anti-HCV compounds and the tissue containing the carboxylesterases responsible for the biotransformation. For example, a specific inhibitor for intestinal carboxylesterase would potentially increase oral absorption and liver exposure of the anti-HCV compound by preventing its degradation during passage through the intestinal epithelium to the portal vein. Specific inhibitors of plasma and blood esterases will stabilize the compounds present in the circulatory system, which may increase the exposure of the drug to the liver, a target organ for anti-HCV therapy (Xie, et al., *supra;* Satoh, T., et al., Drug Metab. Dispos. 30(5):488-493 (2002); and references therein).

Use of an inhibitor specific for liver carboxylesterases would also potentially increase liver exposure, as well as systemic exposure to the drug. Colonic administration should enhance intestinal absorption of the anti-HVC compound because of low levels carboxylesterases in the colon, and decreased first-pass effect in the liver, since only a part of colonic circulation is directed to the portal vein.

In the compositions and uses disclosed herein, a single inhibitor compound may be used, such as a general inhibitor for inhibiting multiple carboxylesterase activities. In another embodiment, a single relatively specific inhibitor, preferentially acting on a specific carboxylesterase isozyme, is used. In some embodiments, multiple carboxylesterae inhibitors may be used. Combinations include different general inhibitors, for example, to target a larger number of carboxylesterases than may not be inhibited by a single broad spectrum inhibitor, a combination of a general inhibitor and a specific inhibitor, for example when several esterases act upon the anti-HCV compounds but certain isozymes predominate in the cleavage reaction; and combinations of specific inhibitors, for example to target specific carboxylesterase isozymes.

The suitability of a carboxylesterase inhibitor may be confirmed by the guidance provided herein and by methods well known in the art. In one embodiment, *in vitro* systems may be used. For example, body fluids (*e.g*., blood), tissue homogenates, or microsomal preparations from tissues (*e.g.*, liver, intestine, etc.) are contacted with the anti-HCV compound in presence or absence of varying concentrations of inhibitor. Decrease in substrate concentration and/or accumulation of metabolic product (*e.g.,* aniline metabolite) are measured by techniques that include, by way of example and not limitation, LC/MS/MS, CC/MS and/or HPLC. These initial determinations can provide an IC₅₀ of the carboxylesterase inhibitor sufficient to affect biotransformation of the anti-HCV compounds. Moroever, use of purified carboxylesterase enzymes, cDNA expressed enzymes, cells transfected with cloned carboxylesterase genes, etc., can be used to identify the specific enzymes responsible for the biotransformation.

Effectiveness of the inhibitors may be further tested through the use of cell, tissue, organ culture systems, or organ perfusion methods (see, *e.g.,* Gebhardt, R. et al., Drug Metab. Rev. 35(2-3):145-213 (2003); Chow, F.S. et al., Drug Metab. Dispos. 25(5):610-6 (1997); Ogawara, K et al., J. Drug Target.;6(5):349-60 (1999)). For cell culture systems, the cells may be derived from the organs being targeted for delivery of the inhibitor, such as the liver or intestine (see, *e.g.,* Gebhardt, *supra;* Tavelin, S., J. Pharmacol. Exp. Ther. 290(3):1212-21 (1999).

Finally, *in vivo* effectiveness of the carboxylesterase inhibitors may be tested in a variety of suitable experimental organisms, for example, mammalian organisms, including, but not limited to, rodents, such as mouse and rats; lagomorphs; ungulates; and primates (*e.g*., monkeys, chimpanzees, etc.).

Generally, active inhibitory compounds are those that exhibit an IC₅₀ (*e.g.,* concentration of inhibitor that yields a 50% reduction in metabolic conversion of anti-HCV compound) in the particular assay in the range of about 1 mM or less. Carboxylesterase inhibitors which exhibit an IC₅₀, for example, in the range of about 500 µM,100 µM, 10 µM, 5 µM,1 µM, 100 nM, 10 nM, 1 nM, or even lower, will be useful for as therapeutics in combination with the anti-HCV compounds. Generally, an inhibitor plasma concentration of 5 µM or less is a reasonable IC₅₀ for *in vivo* applications.

In addition to methods of confirming the suitability of a carboxylesterase inhibitor, the present disclosure further describes methods of identifying anti-HCV hydrolase inhibitors to identify other inhibitor compounds. In some embodiments, the method comprises contacting a system containing an anti-HCV hydrolase with a candidate compound, and determining whether the candidate compound inhibits hydrolysis of the compound. Systems containing an anti-HCV hydrolase include any of the *in vitro* and *in vivo* systems described above.

Changes in substrate concentration and appearance of metabolic product are detected using the methods described. Candidate compounds can range from small organic molecules to large polymers and biopolymers, and can include, by way of example and not limitation, small organic compounds, saccharides, carbohydrates, polysaccharides, lectins, peptides and analogs thereof, polypeptides, proteins, antibodies, oligonucleotides, polynucleotides, nucleic acids, etc. In one embodiment, the candidate compounds screened are small organic molecules having a molecular weight in the range of about 100-2500 daltons. Such candidate molecules will often comprise cyclical structures composed of carbon atoms or mixtures of carbon atoms and one or more heteroatoms and/or aromatic, polyaromatic, heteroaromatic and/or polyaromatic structures. The candidate agents may include a wide variety of functional group substituents. In one embodiment, the substituent(s) are independently selected from the group of substituents known to interact with proteins, such as, for example, amine, carbonyl, hydroxyl and carboxyl groups..

Various art-known approaches may be used to synthesize libraries of compounds, such as small organic compounds, peptides and/or peptide analogs, for screening. Non-limiting examples of solid-phase and solution phase chemical synthesis strategies and conditions useful for synthesizing combinatorial libraries of small organic and other compounds may be found in Bunin, The Combinatorial Index, Academic Press, London, England (1998) (see, e.g., Chapter 1-6) and Hermkens et al., Tetrahedron 52:4527-4554 (1996); WO 95/02566; U.S. Patent No. 5,962,736; U.S. Patent No. 5,766,481; U.S. Patent No. 5,736,412 and U.S. Patent No. 5,712,171.

### 6.4 Sustained/Delayed Release Pharmaceutical Compositions of Anti-HCV Compounds.

As described above, another basis for protecting a compound with a haloalkylamide moiety from metabolic transformation is formulating the compounds as compositions that limit the exposure of the compounds to hydrolase activity. As noted above, carboxylesterase activities that may be responsible for biotransformation of the anti-HCV compounds are higher in the stomach as compared to the small intestine or large intestine. Differences in carboxylesterases activities are also found within the small intestine itself, with the ileum (the final section of the small intestine) displaying lower levels of hCE-1 while the duodenum, a hollow jointed tube that forms the first part of the small intestine and connects the stomach to the jejunum (the middle part of the small intestine), displays lower levels of hCE-2 activity. This differential expression is also seen for the intestinal carboxylesterase identified by Schwer, H et al., Biochem Biophys Res Commun. 233(1):117-20 (1997)), as described above. For this carboxylesterase, the colon and rectum display lower levels of expression than the small intestine, and within the small intestine, highest expression occurs in the jejunum compared to duodenum and ileum.

By formulating the anti-HCV compounds as compositions that preferentially release the compounds in the small intestine and/or large intestine, hydrolysis may be minimized, thus enhancing compound uptake and bioavailability. Selective release may be based on differential pHs in the environment of the stomach, small intestine, and large intestine, and/or by controlling the time or delay in release of the anti-HCV compounds. To bypass the stomach, which has an acidic pH, the pharmaceutical compositions may be formulated to be soluble at pH 5.5 and above. Within the small intestine, the environment of the duodenum is a pH of about 6 to about 6.5 while the environment of the jejunum and ilium is a pH of about 7.0 to about 8.0. Since the small intestine is the main site of absorption for nutrients and many types of drugs, compound release may be targeted based on pH differences within the small intestine and the transit time required for passage of the injested material through the digestive system. Delivery may be similarly targeted to the colon, which has a pH of about 5.5 to about 7.0.

Various orally administered compositions maybe made to selectively release the anti-HCV compounds. In some embodiments, the compounds are formulated as microparticles, microcapsules, microspheres or nanoparticles prepared in sustained release or delayed release form. The compositions may be biodegradable or non-biodegradable (see, *e.g.,* "Microencapsulates: Methods and Industrial Applications," in Drugs and Phamaceutical Sciences, Vol 73 (Benita, S. ed.) Marcel Dekker Inc., New York, (1996); Mathiowitz, et al., J. Appli. Polymer Sci. 35:755-774 (1988); incorporated herein by reference). As used herein, microparticles, microspheres, microcapsules and nanoparticles refer to a particle, which is typically a solid and/or polymeric particle, containing the substance to be delivered. The substance is within the core of the particle or embedded within the particle's polymer network. Generally, the difference between microparticles, which encompass microcapsules and microspheres, and nanoparticles is one of size. Typically, microparticles have a particle size range of about 1 to about >1000 µm. Nanoparticles have a particle size range of about 10 to about 1000 nm. Preferably, the sustained release compositions are microparticles between about 1 µm and about 1000 µm, more preferably between about 2 µm to about 300 µm size. Shape of the microparticles and nanoparticles may be spherical or irregular.

In some embodiments, the microparticles or nanoparticles are formulated to be stable in the stomach but disintegrate in the small intestine and/or large intestine. The rate of release of the drug is determined by both the nature of the drugs (*e.g.,* anti-HCV compound and/or carboxylesterase inhibitor) and by the drug/polymer ratio, polymer structure, and properties of the capsule shell. The nature of the microcapsule shell, in turn, can be predetermined or constructed, as is known in the art, by selection of the type and quantity of polymer and the conditions under which the shell is formed. In some embodiments, the microcapsule shell thickness is between about 0.01 µm to about 90 µm. In other embodiments, the shell thickness is between about 2 µm to about 30 µm.

A variety of polymers may be useful for making the microparticles and nanoparticles containing the anti-HCV compounds. In some embodiments, the anti-HCV compounds are microencapsulated by an enteric polymer. As further described below, suitable enteric polymers include methacrylic acid/methyl methacrylate copolymer, methacrylic acid/ethyl acrylate copolymer, methacrylic acid/methyl acrylate/methyl methacrylate copolymer polyacrylic acid, polyacrylate, polyacrylamide, ethyl cellulose, hydroxypropyl cellulose, hydroxypropylmethyl cellulose phthalate, carboxymethyl cellulose, polyvinyl acetate phthalate, carboxymethylethyl cellulose, shellac, acrylic resins, acetate succinate, cellulose acetate phthalate, cellulose acetate trimellitate, or compatible combinations thereof. Other enteric polymers are known to the skilled artisan and are within the scope of the present disclosure.

In some embodiments, the microparticles and microcapsules are based on biodegradable or bioerodable polymers. As used herein, "bioerodable" refers to a material that will, at least in part, dissolve, degrade or erode in the fluid environment of use. In some embodiments, the microparticles are those based on methacrylic acid/methyl methacrylate copolymers, methacrylic acid/ethyl acrylate copolymers, and methacrylic acid/methyl acrylate/methyl methacrylate copolymers, available under the tradename Eudragit® (Rohm Pharma Co.) (see, *e.g.,* U.S. Patent No. 5,401,512). The Eudragit L series is a copolymer of methacrylic acid and methyl methacrylate of which the molar proportion of the monomer units is at a ratio of about 1:1. Various L series polymers include, among others, Eudragit L 12.5, Eudragit L 12.5P, Eudragit L100, Eudragit L 100-55, Eudragit L-30, Eudragit L-30 D-55. The Eudragit S series is a copolymer of methacrylic acid and methyl methacrylate of which the molar proportion of the monomer units is at a ratio of about 1:2. Various S series polymers include, among others, Eudragit S 12.5, Eudragit S 12.5P, and Eudragit S100. The Eudragit RL series is a tripolymer of ethyl acrylate, methyl methacrylate, trimethylammonioethyl methacrylate chloride of which the molar proportion of the monomer units is at a ratio of about 1:2:0.2. Various Eudragit RL series include, among others, Eudragit RL 12.5, Eudragit RL 100, Eudragit RL PO, Eudragit RL 30D. The Eudragit RS series is a tripolymer of ethyl acrylate, methyl methacrylate, and trimethylammonioethyl methacrylate chloride of which the molar proportion of the monomer units is at a ratio of about 1:2:0.1. Various polymers of the Eudragit RS series include, among others, Eudragit RS 12.5, Eudragit RS 100, Eudragit RS PO, and Eudragit RS 30D. The Eudragit NE series is a neutral copolymer of ethyl acrylate and methyl methacrylate of which the molar proportion of the monomer units is at a ratio of about 2:1. Eudragit NE series include Eudragit NE 30D and Eudragit NE 40D. The polymers above may be used alone, or as compatible mixtures thereof.

In other embodiments, the pharmaceutical compositions of the anti-HCV compounds are microcapsules prepared in an anionic copolymer of methacrylic acid and methyl methacrylate available under the tradename Eudragit L. Eudragit L 30 D-55 may be used to form pharmaceutical compositions solubilizing above pH 5.5 for targeted drug delivery in the duodenum. Similarly, Eudragit L 100 is soluble above pH 6.0 and may be used for formulations for targeted drug delivery in the duodenum or colon.

In other embodiments, the pharmaceutical compositions of the anti-HCV compounds are microcapsules prepared in a copolymer of methacrylic acid and methyl methacrylate, available under the tradename Eudragit S. Eudragit S 100 may be used to form compositions solubilizing above pH 7.0 for targeted drug delivery in the ileum or colon.

In still other embodiments, the pharmaceutical compositions of the anti-HCV compounds are microcapsules prepared in mixtures of methacrylic acid and methyl methacrylate polymers, such as admixtures of Eudragit L100 and Eudragit S100. The admixture of the Eudragit S and the Eudragit L may be at various ratio to generate the desired dissolution characteristics. Admixtures of Eudragit S and Eudragit L typically solubilize at pH of about 6 to about 7. In some embodiments, a suitable ratio of Eudragit S to Eudragit L is about 5:1, 2:1, 1:1.1:2, or 1:5. Other ratios of the polymers may be chosen to have the desired release characteristics as described herein.

In some embodiments, the microparticles and microcapsules may be based enteric polymers of polysaccharides and derivatives thereof. Suitable polysaccharides and polysaccharide derivatives include, by way of example and not limitation, alkyl cellulose, hydroxyalkyl cellulose, cellulose ether, cellulose esters, methyl cellulose, ethyl cellulose, hydroxypropylmethyl cellulose, carboxyethylcellulose, cellulose acetate, cellulose acetate phthalate, hydroxypropylmethyl cellulose phthalate, starch or starch derivatives (*e.g.*, poly(acryloyl hydroxyethyl) starch (see, *e.g.,* US Patent No. 5,391,696; 6,703,048), and compatible mixtures thereof.

Other biodegradable polymers for encapsulating the compounds may comprise synthetic polymers such as polylactic acid, polyglycolic acid, polylactic acid-glycolic acid, polylactide (PLA), polyglycolide (PGA), poly(lactide-co-glycolide) (PLGA), poly(caprolactone), polyanhydrides, polydioxanone trimethylene carbonate, polyhybroxyalkonates (*e.g.,* poly(β-hydroxybutyrite)), poly(γ-ethyl glutamate), poly(DTH iminocarbony (bisphenol A iminocarbonate), poly (ortho ester), polethyleoxide, polyurethanes, polycyanoacrylates (*e.g.,* poly(isobutylcyanoacrylate), and compatible blends and mixtures thereof. Microparticles may also be based on biopolymers such as fibrin, casein, serum albumin, collagen, gelatin, lecithin, chitosan, alginate or poly-amino acids such as poly-lysine., and compatible mixtures thereof.

In some embodiments, release modifiers may be added to the compositions to modify the compound release properties. This may be useful when the compound has reduced solubility in the higher pH environment of the small intestine or if changes to the release rate is desired. Release modifiers include, among others, surfactants and emulsifiers, such as sodium lauryl sulfate, Tween 20, and sodium lauryl sulfate.

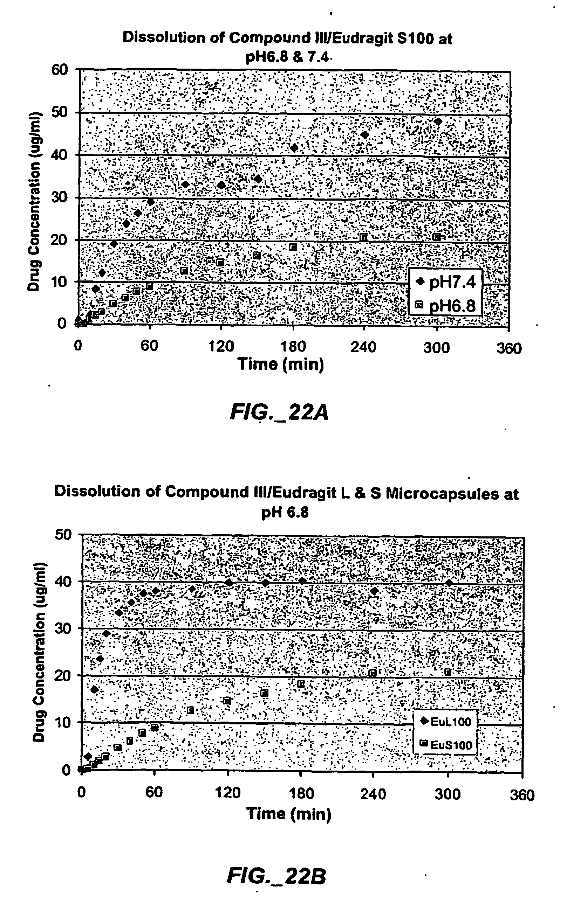

The pharmaceutical compositions of the microparticles and microcapsules disclosed herein may be made to have a characteristic release profile, *e.g.*, dissolution profile that will provide optimal drug uptake and stability, such as by targeted delivery that reduces hydrolysis of the anti-HCV compounds by carboxylesterases. As is known in the art, a rate corresponding to a release time of 1 to 50 hrs or 5 to 10 hrs may be used as a guide for forming the compositions when targeting delivery to the small intestine, while a release time of 25-50 hrs may be used when targeting delivery to the large intestine. The rate may be measured *in vitro* at a defined physiological temperature (*e.g.,* 37°C) in a solution approximating or simulating the environment of the intestine being targeted. The release time may also account for the movement of the composition through the intestine. In some embodiments, the microcapsule compositions may be made to release the anti-HCV compound at the release profiles shown in FIGS. 22A and 22B. Thus, in some embodiments, the microcapsule compositions release less than about 10% or less than about 5% of the compound after 2 hrs in a gastric environment; release from about 10 to about 50% or about 20% to about 50% of the compound after two hours in an intestinal environment; release from about 30% to about 80% or about 40% to about 70% after about 4 hrs in the an intestinal environment; release from about 40% to about 90% or about 60% to about 90% after about 8 hrs in an intestinal environment; and release more than about 80% after 12 hrs in an intestinal environment. It is to be understood that the release times may be adjusted to preferentially target the anti-HCV compound to specific regions of the intestine.

When preferentially targeting the anti-HCV compounds to the colon, the microcapsule release times may be adjusted to slower release times so that more of the compounds are released in the colon rather than the small intestine. In some embodiments, the microparticle composition may be formulated to release not more than about 10% or less than about 5% of the compound after 2 hrs in a gastric environment; release from about 5% to about 30% or about 10% to about 30% of the compound after four hours in an intestinal environment; release from about 20% to about 60% or about 30% to about 50% after about 12 hrs in the an intestinal environment; release from about 40% to about 90% or about 50% to about 80%, after about 24 hrs in an intestinal environment; and release less than about 80% after 48 hrs in an intestinal environment.

Various methods for making microparticles and microcapsules containing the subject compounds are well known in the art, including solvent diffusion (see for example, U.S. Patent No. 4,389,330; U.S. Patent No. 4,272,398); emulsification-evaporation (Maysinger, D. et al., Exp. Neuro. 141: 47-56 (1996); Jeffrey, H. et al., Pharm. Res. 10: 362-68 (1993)), spray drying, and extrusion methods. As noted above, the anti HCV compounds may be made as microparticles, and optionally encapsulated in bio-degradable polymers and/or diffusion barriers, such as the enteric polymers described herein (*e.g.*, Eudragit NE30, ethycellulose emulsion) to modify the release characteristics. Methods for making nanoparticles are similar to those for making microparticles and include, by way of example and not limitation, emulsion polymerization in continuous aqueous phase, emulsification-evaporation, solvent displacement, and emulsification-diffusion techniques (see, *e.g.*, Kreuter, J., "Nano-particle Preparation and Applications," in Microcapsules and Nanoparticles in Medicine and Pharmacy, M. Donbrow, ed., pg. 125-148, CRC Press, Boca Rotan, FL, 1991; incorporated herein by reference).

In some embodiments, the microparticles and microcapsules may be made by an emulsion/solvent diffusion process. Generally, in this method, the polymer along with the compound may be dissolved in a volatile organic solvent, or the compound, either in soluble or particulate form, is added to a polymer dissolved in the organic solvent. The organic solvent may be a mixture of organic solvents, such as for example, ethanol, isopropanol and dichloromethane. In an exemplary embodiment, the ratio of ethanol, isopropanol and dichloromethane is about 8 to about 2 to about 5. The mixture is then suspended in an aqueous phase containing a surface active agent, such as poly(vinyl alcohol) or Tween, and stirred, vortexed, or homogenized to produce a water-oil emulsion. Microparticles are collected by filtration. Alternatively, the solvent is evaporated to leave solid microparticles. Microparticles of various sizes, *e.g.,* about 1 µm to about 1000 µm and of various morphologies may be obtained by this method. Preferably the microparticles are microcapsules between about 1 µm and about 1000 µm, and more preferably between about 2 µm and about 300 µm.

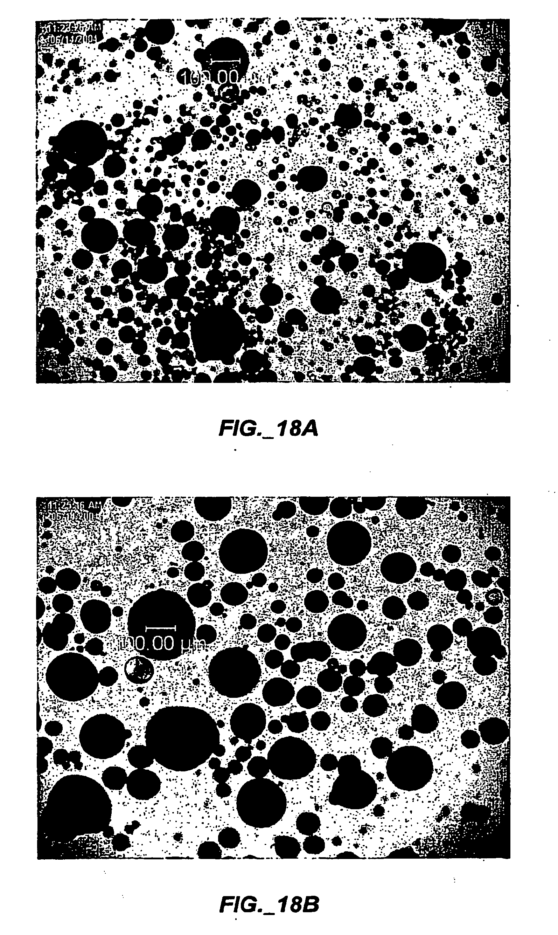

A number of factors influencing the microparticle produced by solvent diffusion or other emulsification procedures include rate of stirring, temperature, and polymer to drug ratio. Generally, the rate of stirring and the size of microparticles formed are directly correlated. The greater the rate of stirring, the smaller the size and the size distribution of the formed microcapsules. For example, 250 rpm in 1% polyvinyl alcohol solution, results in the formation of microcapsules which range in size from about 5 µm and about 250 µm (see FIGS. 18A and 18B).

Temperature is also a significant factor when forming microcapsules. High temperatures lead to microcapsules with thinner and sticky shells due to increased rate of alcohol diffusion into the aqueous phase. Low temperatures decrease phase separation by increasing solution viscosity and decreasing rate of alcohol diffusion rate into the aqueous phase. For the exemplary microcapsules described herein, the optimum temperature for the microcapsule preparation is between about 20 °C to about 28 °C.

Another important factor is the polymer to drug ratio: the higher the ratio, the thicker the polymer shell and the smaller the drug loading. In some embodiments, the polymer to drug ratio ranges from about 10:1 to about 10:5, preferably 10:3 to about 10:5. Exemplary polymer to drug ratios include, for example, about 10:3, about 10:3.5 or about 10:4.5.

In other embodiments, the microparticles are made by a spray drying method. This method typically involves dissolving a the polymer and compound in an appropriate solvent, dispersing a solid or liquid active agent into the polymer solution, and then spray drying the polymer solution to form microparticles. The method of spray drying a solution of a polymer and compound refers to a process wherein the solution is atomized to form fine droplets and dried by direct contact with carrier gases. The size of the particulates of polymer solution is controlled by of the characteristics of the nozzle used to spray the polymer solution, nozzle pressure, the flow rate, the polymer used, the polymer concentration, the type of solvent and the temperature of spraying, and polymer molecular weight.

Generally, the solvent for forming the microparticles is selected based on solubility of the polymer, biocompatibility, and suitability for the compound or composition being delivered. Where appropriate, organic solvents used to dissolve the polymer typically are volatile, have a low boiling point, or are removable under vacuum. Useful organic solvents included, by way of example and not limitation, methanol, ethanol, isopropanol, dichloromethane, methylene chloride, ethyl acetate, acetone, acetonitrile, choroform, and compatible combinations thereof.

The present disclosure also describes a method of preparing microcapsules of the anti-HCV compounds. In general, the method comprises (a) dissolving an anti-HCV compound and the polymer in a solvent to form a mixture, (b) forming an emulsion with the mixture and an aqueous solution, (c) generating the microparticles or microcapsules in the solution mixture (*e.g.,* by stirring, vortexing, or homogenzing), and (d) collecting the microparticles or microcapsules. Compositions obtained by the method may be processed through additional steps, including, among others, drying to evaporate off the solvent, washing to remove residual contaminants, screening for particles of a defined size range or distribution, and separating or isolating particles of a defined size range (*e.g.,* microparticle and nanoparticles). Drug loading of the compositions may be determined by methods known to the skilled artisan, including by way of example and not limitation, high performance liquid chromatography (HPLC), immunochemistry using drug specific antibodies, gas chromatography (GC), mass spectroscopy (MS), GC-MS, spectrophotometry, and polarography.

Other excipients and additives may be added to the microparticle and microcapsule compositions, as further described below. The pharmaceutical compositions comprising the encapsulates of the anti-HCV compound may be compounded in capsules or in tablets. The capsule or tablets may be formulated to disintegrate in the stomach, releasing the microencapsulates, which will pass through the acidic environment of the stomach when formulated to be soluble at pH 5.5 and above and then begin to release the active compound as they enter the small intestine. Further, capsules or tablets may also have an enteric coating, as described below, to increase the time required for dissolution and provide additional delay time between administration of the composition and release of the anti-HCV compounds.

Although the descriptions provide for microparticles and microcapsules of anti-HCV compounds, it is to be understood that the compositions may also be used to deliver the carboxylesterase inhibitors, either alone or in combination with the anti-HCV compounds. Compositions containing both the compound and inhibitor may be used for simultaneous administration, where the inhibitor is released at the site where the compound is also released. In other embodiments, the compound and inhibitor are prepared separately, and administered separately to provide a dose of the inhibitor, such as prior to administration of the compound.

### 6.5 Other Pharmaceutical Compositions

In addition to the above, provided herein are other pharmaceutical compositions comprising an anti-HCV compound and/or a carboxylesterase inhibitor for the prevention or treatment of HCV infection. The compounds and inhibitors may be formulated in pharmaceutical compositions *per se,* or in the form of a hydrate, solvate, or pharmaceutically suitable salts thereof. Accordingly, in one embodiment, the pharmaceutical compositions comprise a pharmaceutically acceptable carrier and a pharmacologically effective amount of an anti-HCV compound and/or a carboxylesterase inhibitor

As described above, pharmaceutically acceptable salts of the anti-HCV compounds and/or carboxylesterase inhibitors are intended to include any art recognized pharmaceutically acceptable salt of the compound or inhibitor which is made with counterions understood in the art to be generally acceptable for pharmaceutical uses and which possesses the desired pharmacological activity of the parent compound. Examples of salts include sodium, potassium, lithium, ammonium, calcium, as well as primary, secondary, and tertiary amines, esters of lower hydrocarbons, such as methyl, ethyl, and propyl. Other salts include organic acids, such as acetic acid, propionic acid, pyruvic acid, maleic acid, succinic acid, tartaric acid, citric acid, benzoic acid, cinnamic acid, and salicylic acid. Other salt forms will be known to the skilled artisan.

As used herein, pharmaceutically acceptable vehicle or pharmaceutically acceptable carrier comprise any of standard pharmaceutically accepted carriers used by those skilled in the art for administering a pharmaceutical composition. Thus, the anti-HCV compounds and/or carboxylesterase inhibitors may be prepared as formulations in pharmaceutically acceptable excipients suitable for any mode of administration.

For oral administration, the pharmaceutical compositions may be prepared with pharmaceutically acceptable excipients such as binding agents (*e.g.,* starch, carboxymethyl cellulose, hydroxylpropyl methyl cellulose), fillers (*e.g.,* lactose, microcrystalline cellulose, calcium phosphate, etc.), lubricants (*e.g.*, magnesium stearate, talc, silicon dioxide, etc.); disintegrants (potato starch and sodium starch glycolate); or wetting agents (*e.g*., sodium lauryl sulfate). Formulations for oral administrations may take various forms, including, but not limited to, tablets, capsules, lozenges, and powders. Suitable tablet formulations, such as for the microcapsules described herein, include, for example, Avicel pH101, Ac-Ti-Sol, stearic acid, 316 Fast Flo Lactose, or compatible combinations thereof.

Capsules may be made from various materials, including, by way of example and not limitation, gelatin or polysaccharides (*e.g*., starch, agar, pectin, hydroxypropyl methycellulose, hydroxyethycellulose, etc.), or mixtures thereof (see, *e.g*., U.S. Patent 6,319,518). The capsule compositions may also include a plasticizer, such as glycerin, triacetin, sorbitol, polyethylene glycol, propylene glycol, citrate, and phthalate, to impart form and flexibility where desired. Capsules from non-gelatin substitute, carrageen, are described in U.S. Patent No. 6,214,376. Capsule materials are chosen to be compatible with the fill material, for example, microcapsules. Capsules based on gelatin may use gelatin derived from animal skin by hydrolysis with an acid (type A gelatin) or gelatin derived from bones and animal skin by hydrolysis with an alkaline solution (type B gelatin). Exemplary capsules are based on hydroxypropyl methycellulose (HPMC). 0

Pills, tablets, or capsules may have an enteric coating and/or polymer film that remains intact in the stomach but dissolves in the intestine. Various enteric coatings are known in the art, a number of which are commercially available, including the enteric polymers described for preparing microencapsulates. Enteric coatings include, by way of example and not limitation, methacrylic acid/methacrylic acid ester copolymers, polymer cellulose ether, cellulose acetate phathalate, polyvinyl acetate phthalate, and hydroxypropyl methyl cellulose phthalate. Polymer films are described in, for example, US Patent No. 6,309,666.

In other embodiments, the anti-HCV compounds and/or the carboxylesterase inhibitors may be in liquid form prepared in diluents for administration orally or by injection. These diluents include, by way of example and not limitation, saline, phosphate buffer saline (PBS), aqueous ethanol, or solutions of glucose, mannitol, dextran, propylene glycol, polyethylene glycol (*e.g.*, PEG400), and mixtures thereof. Suitable diluents also include nonaqueous vehicles, including oils and other lipophilic solvents, such as various vegetable oils, animal oils, and synthetic oils (*e.g*., peanut oil, sesame oil, olive oil, corn oil, safflower oil, soybean oil, etc.); fatty acid esters, including oleates, triglycerides, etc.; cholesterol derivatives, including cholesterol oleate, cholesterol linoleate, cholesterol myristilate, etc.; liposomes; and the like. The diluents may also contain suspending agents (*e.g.*, soribitol solution, cellulose derivatives, or hydrogenated edible fats) and emulsifying agents (*e.g.,* lecithin or acacia).

Formulations for rectal or vaginal administration may be in the form of salves, tinctures, crèmes, suppositories, enemas or foams. Suppositories for rectal application may contain conventional suppository bases such as cocoa butter, carbowaxes, polyethylene glycols, or glycerides, which are solid or semi-solid at room temperature but liquid at body temperature.

Formulations for administration by inhalation or insufflation may be in the form of powders or liquid with an excipient for delivery in the form of a spray from pressurized packs or a nebulizer with a suitable propellant. Capsules or cartridges for use in an inhaler or insufflator may be formulated containing a powdered mixture of the compound with a suitable base such as lactose or starch.

Additionally, the pharmaceutical compositions may include bactericidal agents, stabilizers, buffers, emulsifiers, preservatives, flavoring, sweetening agents, coloring agents, dyes and the like as needed or desired in the various formulations.

The pharmaceutical compositions comprising the anti-HCV compound and/or carboxylesterase inhibitor may be manufactured in a manner known to the skilled artisan, such as by conventional means of mixing, dissolving, granulating, levigating, emulsifying, encapsulating, entrapping, suspending, or lyophilizing processes. Suitable pharmaceutical formulations and methods for preparing such compositions may be found in various standard references, such as Remington's Pharmaceutical Sciences, 17th edition, Mack Publishing Co., Philadelphia, PA (1985) and Handbook of Pharmaceutical Excipients, 3rd Ed, Kibbe, A.H. ed., Washington DC, American Pharmaceutical Association (2000); hereby incorporated by reference in their entirety.

Additionally, the anti-HCV compounds and/or carboxylesterase inhibitors, either separately or as a combination, may also be introduced or encapsulated into the lumen of liposomes for delivery and for extending life time of the compounds. As known in the art, liposomes can be categorized into various types: multilamellar (MLV), stable plurilamellar (SPLV), small unilamellar (SUV) or large unilamellar (LUV) vesicles. Liposomes can be prepared from various lipid compounds, which may be synthetic or naturally occurring, including phosphatidyl ethers and esters, such as phosphotidylserine, phosphotidylcholine, phosphatidyl ethanolamine, phosphatidylinositol, dimyristoylphosphatidylcholine; steroids such as cholesterol; cerebrosides; sphingomyelin; glycerolipids; and other lipids (see, *e.g.*, U.S. Patent No. 5,833,948).

Cationic lipids are also suitable for forming liposomes. Generally, the cationic lipids have a net positive charge and have a lipophilic portion, such as a sterol or an acyl or diacyl side chain. Preferably, the head group is positively charged. Typical cationic lipids include 1,2-dioleyloxy-3-(trimethylamino)propane; N-[1-(2,3,-ditetradecycloxy)propyl]-N,N-dimethyl-N-N-hydroxyethylammonium bromide; N-[1-(2,3-dioleyloxy)propyl]-N,N-dimethyl-N-hydroxy ethylammonium bromide; N-[1-(2,3-dioleyloxy) propyl]-N,N,N-trimethylammonium chloride; 3-[N-(N',N'-dimethylaminoethane) carbamoyl] cholesterol; and dimethyldioctadecylammonium.

Of interest are fusogenic liposomes, which are characterized by their ability to fuse with a cell membrane upon appropriate change in physiological condition or by presence of fusogenic component, such as a fusogenic peptide or protein. In some embodiments, the fusogenic liposomes are pH and temperature sensitive in that fusion with a cell membrane is affected by change in temperature and/or pH (see for example, U.S. Patent No. 4,789,633 and 4,873,089). Generally, pH sensitive liposomes are acid sensitive. Thus, fusion is enhanced in physiological environments where the pH is mildly acidic, for example the environment of a lysosome, endosome and inflammed tissues (*e.g.*, cirrhotic liver). This property allows direct release of the liposome contents into the intracellular environment following endocytosis of liposomes (Mizoue, T., Int. J. Pharm. 237: 129-137 (2002)).

Another form of fusogenic liposomes comprises liposomes that contain a fusion-enhancing agent. When incorporated into the liposome or attached to the lipids, the agents enhance fusion of the liposome with other cellular membranes, thus resulting in delivery of the liposome contents into the cell. The agents may be fusion enhancing peptides or proteins, including hemaggulutinin HA2 of influenza virus (Schoen, P., Gene Ther. 6: 823-832 (1999)); Sendai virus envelope glycoproteins (Mizuguchi, H., Biochem. Biophys. Res. Commun. 218: 402-407 (1996)); vesicular stomatitis virus envelope glycoproteins (VSV-G) glycoprotein (Abe, A. et al., J. Virol. 72: 6159-63 (1998)); peptide segments or mimics of fusion enhancing proteins; and synthetic fusion enhancing peptides (*e.g*., Kono, K. et al., Biochim. Biophys. Acta. 1164: 81-90 (1993); Pecheur, E.I., Biochemistry 37: 2361-71 (1998); and U.S. Patent No. 6,372,720).

Liposomes also include vesicles derivatized with a hydrophilic polymer, as provided in U.S. Patent No. 5,013,556 and 5,395,619, hereby incorporated by reference, (see also, Kono, K. et al., J. Controlled Release 68: 225-35 (2000); Zalipsky, S. et al., Bioconjug. Chem. 6: 705-708 (1995)) to extend the circulation lifetime *in vivo.* Hydrophilic polymers for coating or derivation of the liposomes include polyethylene glycol, polyvinylpyrrolidone, polyvinylmethyl ether, polyaspartamide, hydroxymethyl cellulose, hydroxyethyl cellulose, and the like. In addition, as described above, attaching proteins that bind a cell surface protein which is endocytosed, *e.g.,* capsid proteins or fragments thereof tropic for a particular cell type and antibodies for cell surface proteins that undergo internalization may be used for targeting and/or facilitating uptake of the liposomes to specific cells or tissues.

Liposomes are prepared by ways known in the art (see, *e.g.,* Szoka, F. et al., Ann. Rev. Biophys. Bioeng. 9: 467-508 (1980)). One typical method is the lipid film hydration technique in which lipid components are mixed in an organic solvent followed by evaporation of the solvent to generate a lipid film. Hydration of the film in aqueous buffer solution, preferably containing the subject compounds and compositions, results in an emulsion, which is sonicated or extruded to reduce the size and polydispersity. Other methods include reverse-phase evaporation (see, *e.g.*, Pidgeon, C. et al., Biochemistry 26: 17-29 (1987); Duzgunes, N. et al., Biochim. Biophys: Acta. 732: 289-99 (1983)), freezing and thawing of phospholipid mixtures, and ether infusion.

Hydrogels are also useful in delivering the subject agents into a host. Generally, hydrogels are crosslinked, hydrophilic polymer networks permeable to a wide variety of drug compounds. Hydrogels have the advantage of selective trigger of polymer swelling, which results in controlled release of the entrapped drug compound. Depending on the composition of the polymer network, swelling and subsequent release may be triggered by a variety of stimuli, including pH, ionic strength, thermal, electrical, ultrasound, and enzyme activities. Non-limiting examples of polymers useful in hydrogel compositions include, among others, those formed from polymers of poly(lactide- co-glycolide), poly(N-isopropylacrylamide); poly(methacrylic acid-γ-polyethylene glycol); polyacrylic acid and poly(oxypropylene-co-oxyethylene) glycol; and natural compounds such as chrondroitan sulfate, chitosan, gelatin, or mixtures of synthetic and natural polymers, for example chitosan-poly(ethylene oxide). The polymers are crosslinked reversibly or irreversibly to form gels embedded with the anti-HCV compounds and/or carboxylesterase inhibitors, or pharmaceutical compositions thereof (see, *e.g.,* U.S. Patent No. 6,451,346; 6,410,645; 6,432,440; 6,395,299; 6,361,797; 6,333,194; 6,297,337 Johnson, O. et al., Nature Med. 2: 795 (1996);

Another pharmaceutical compositions may include those in the form of transdermal patches for delivery of the compounds through the skin by diffusion or electrically mediated transport (see, *e.g.,* Banga, A.K. et al., Int J Pharm. 179(1):1-19 (1999); U.S. Patent Nos. 5,460,821, 5,645,854, 5,853,751, 6,635,274,6,564,093).

### 6.6 Kits

The compositions disclosed herein may be provided in the form of a kit or packaged formulation. A kit or packaged formulation as used herein includes one or more dosages of an anti-HCV compound, and/or one or more carboxylesterase inhibitors, in a container holding the dosages together with instructions for simultaneous or sequential administration to a subject. For example, the package may contain the anti-HCV compounds and/or carboxylesterase inhibitors along with a pharmaceutical carrier combined in the form of a powder for mixing in an aqueous solution, which can be ingested by the afflicted subject. Another example of packaged drug is a preloaded pressure syringe, so that the compositions may be delivered intravenously, intramuscularly, or colonically. Capsules and tablets may be enclosed in bottles or other dispensers, such as blister packs. The kit includes appropriate instructions, which encompasses diagrams, recordings (*e.g.,* audio, video, compact disc), and computer programs providing directions for use of the combination therapy.

### 6.7 Administration

Administration of the anti-HCV compounds, carboxylesterase inhibitors, or compositions thereof can be done in a variety of ways known in the art, including, but not limited to, oral, topical, transdermal, cutaneous, subcutaneous, intravenous, intraperitoneal, intramuscular, nasal, intrathecal, transdermal, vaginal, buccal, and rectal (*e.g.,* colonic administration) delivery. Choosing the appropriate route of administration is well within the skill of the art.

In some embodiments, the method of administration is by oral delivery in the form of a powder, tablet, pill, or capsule, typically formulated with a suitable excipient. Alternatively, oral delivery is by administration of the compounds and compositions prepared in a suitable diluent in the form of a liquid (*e.g.,* syrups, slurries, suspensions, etc.) or emulsion.

In other embodiments, administration is done rectally or vaginally. This may use formulations suitable for topical application in the form of salves, tinctures, crèmes, or foams, and for application into the lumen of the intestine, in the form of suppositories, enemas, foams, etc. Rectal administration has the advantage of bypassing the portal circulation and decreasing initial inactivation by the liver, thus resulting in rapid systemic distribution. In addition, the rectal mucosa is also more capable than the gastric mucosa of tolerating various drug-related irritations.

In further embodiments, administration is by inhalation or insufflation of the compounds and compositions. Delivery may be in the form of an atomized powder or liquid. In the case of a pressurized aerosol, the dosage unit may be determined by providing a valve to deliver the metered amount.

In some embodiments, the administration is carried out cutaneously, subcutaneously, intraperitonealy, intramuscularly, or intravenously. The compound and/or inhibitor may be prepared as injectable preparations, dissolved or suspended in suitable aqueous or lipophilc diluent, as described above, and injected by the appropriate route. The compositions for injection may be prepared directly in a lipophilic solvent or preferably, as emulsions (see, *e.g.,* Liu, F. et al., Pharm. Res. 12: 1060-1064 (1995); Prankerd, R.J. J. Parent. Sci. Tech. 44: 139-49 (1990); and U.S. Patent No: 5,651,991).

Another method of administration is by topical application of the compounds and compositions. These may be applied in the form of solutions, gels, cremes, ointments, creams, suspensions, etc. as is known in the art.

The delivery systems also include sustained release or long-term delivery methods, which are well known to those skilled in the art. Generally, sustained release refers to pharmaceutical compositions or administration methods which allow uniform delivery of the drug for about 8 hrs or longer. Sustained or long term release systems may comprise implantable solids or gels containing the subject compounds or compositions, such as biodegradable polymers described above; pumps, including peristaltic pumps and fluorocarbon propellant pumps; osmotic and mini-osmotic pumps; and the like. Peristaltic pumps deliver a set amount of drug with each activation of the pump, and the reservoir can be refilled, preferably percutaneously through a port. A controller sets the dosage and can also provides a readout on dosage delivered, dosage remaining, and frequency of delivery. Fluorocarbon propellant pumps utilize a fluorocarbon liquid to operate the pump. The fluorocarbon liquid exerts a vapor pressure above atmospheric pressure and compresses a chamber containing the drug to release the drug. Osmotic pumps (and mini-osmotic pumps) utilize osmotic pressure to release the drug at a constant rate. The drug is contained in an impermeable diaphragm, which is surrounded by the osmotic agent. A semipermeable membrane contains the osmotic agent, and the entire pump is housed in a casing. Diffusion of water through the semipermeable membrane squeezes the diaphragm holding the drug, forcing the drug into bloodstream, organ, or tissue. These and other such implants may useful in treating chronic HCV infection through delivery of the compounds and compositions via systemic (*e.g.*, intravenous or subcutaneous) or localized doses in a sustained, long-term manner.

Various combinations of methods of administration may be used to deliver the anti-HCV compounds and the carboxylesterase inhibitors to a host. In some embodiments, an anti-HCV compound and a carboxylesterase inhibitor are administered together as a composition, such as in a pharmaceutical composition. This simplifies treatment and provides simultaneous inhibition of the carboxylesterase activity.

In other embodiments, the anti-HCV compounds may be administered separately from the carboxylesterase inhibitors. In this format, administration of the compounds and/or pharmaceutical compositions may be through a single route or by several different routes, simultaneously or sequentially. Thus, the anti-HCV compounds may be administered by a first route while the carboxylesterase inhibitors are administered by a second route. The first and second routes may be the same, with the compounds and inhibitors being administered sequentially or simultaneously. Alternatively, the first and second routes may be different, with the compounds being administered sequentially or simultaneously via the different routes. For instance, the anti-HCV compounds are hydrolyzed by esterases present in the intestines, blood, and liver. Thus, if the anti-HCV compounds are administered orally, sequential or simultaneous oral administration of the carboxylesterase inhibitors will limit metabolic transformation by esterases in the intestine, thus reducing inactivation of the anti-HCV compound in the intestine. Additional inhibitors may be administered by a second route, *e.g.,* intravenously to reduce metabolic transformation of absorbed anti-HCV compound by esterases in the blood or liver.

As another example, if administration of the anti-HCV compound is carried out intravenously or rectally, the inhibitors may be given orally if such oral administration results in sufficient absorption and distribution of the inhibitor into the circulatory system or target organ to inhibit esterases in the blood or target organ.

As discussed above, in embodiments where the anti-HCV compounds are administered separately from the carboxylesterase inhibitors, administration may be done sequentially. In one embodiment, the carboxylesterase inhibitors are administered prior to administration of the anti-HCV compound. Pretreatment with the carboxylesterase inhibitor may provide additional time for adsorption and delivery of the inhibitor such that the level of carboxylesterase activity is lowered before subsequent administration of the anti-HCV compounds. Pre-conditioning a host in such a manner may lower the dosage of anti-HCV compounds needed to provide a therapeutic benefit.

### 6.8 Effective Dosages

The concentrations of the anti-HCV compounds and/or carboxylesterase inhibitors to be administered will be determined empirically in accordance with conventional procedures. Generally, for administering the anti-HCV compounds, carboxyesterase inhibitors, and pharmaceutical compositions for therapeutic purposes, the subject formulations are given at a pharmacologically effective dose. A pharmacologically effective amount or pharmacologically effective dose is an amount sufficient to produce the desired physiological effect or amount capable of achieving the desired result, such as for treating the disorder or disease condition, including reducing or eliminating one or more symptoms of the disorder or disease. Thus the compounds and compositions described herein may be administered therapeutically to achieve a therapeutic benefit or prophylactically to achieve a prophylactic benefit. By therapeutic benefit is meant eradication or ameliorating of the underlying disorder being treated, and/or eradication or amelioration of one or more of the symptoms associated with the underlying disorder such that the patient reports an improvement in feeling or condition, nothwithstanding that the patient may still be afflicted with the underlying disorder.

In the case of HCV infections, administration of the compounds and compositions to a patient suffering from HCV infection provides a therapeutic benefit when the HCV infection is eradicated or the rate of viral infection reduced, but also when the patient reports a decrease in fatigue, abdominal pain, fever, loss of appetite and nausea associated with HCV infection. Methods for detecting and monitoring HCV infection include, by way of example and not limitation, biopsies of the liver for progression of liver damage; measuring presence of enzyme alanine aminotransferase, which is released into the bloodstream when liver cells die; viral load tests, generally involving nucleic acid amplification methods, such as polymerase chain reaction and signal-amplification-based "branched DNA" assay to detect and/or quantitate anti-HCV RNA in a blood sample; and immunoassays to detect and measure presence of anti-HCV antibodies, including anti-HCV core antigen (see, *e.g.*, Courouce, A.M. et al., Transfusion 40:1198-1202 (2000)).

The compounds and compositions may be administered prophylactically to a patient at risk of viral infection. These include individuals exposed or thought to be exposed to the virus but who have yet to show signs (*e.g.*, antibodies against anti-HCV) or symptoms of viral infection. Candidates for prophylactic treatment include intravenous drug users, recipients of clotting factors, transplant patients, laboratory technicians, or health care providers known to have been exposed to the virus. Thus, the compounds and compositions may be administered prophylactically to reduce the likelihood of HCV infection.

For any anti-HCVcompounds and/or carboxylesterase inhibitors, and combinations thereof, therapeutically effective dose is readily determined by methods known in the art. Factors to consider in determining an appropriate dose include, but are not limited to, size and weight of the subj ect, the age and sex of the subject, the severity of the symptom, the stage of the disease, method of delivery of the compounds and compositions, half-life of the compounds and inhibitors, and efficacy of the anti-HCV compounds. Stage of the disease to consider includes whether the disease is acute or chronic, and the progressiveness of the disease.

An initial effective dose can be estimated from cell culture assays. For example, because the liver is a primary organ for HCV infection, *in vitro* culture systems using liver cells are suitable for initial determination of an effective dose. The cells may be contacted with an anti-HCV compound in the absence of inhibitor to determine the levels of drug useful for inhibiting anti-HCV viral infection. In addition, the concentration of carboxylesterase inhibitors sufficient to inhibit carboxylesterase activity may be determined using cell-free extracts or cell culture. Subsequently, the lower doses of anti-HCV compound can be tested in presence of different carboxylesterase inhibitors in varying concentrations to determine levels of inhibitor required to limit conversion of the anti-HCV compounds and achieve a concentration of active drug sufficient to affect anti-HCV viral infection. Assays for anti-viral activity may use any indicator of HCV infection, such as viral replication, presence of viral expression products, expression of cellular products in response to HCV infection, and virally induced cell death.

Following *in vitro* studies, a dose can then be formulated in experimental animal models to generate data on circulating concentration or tissue concentration, including that of the IC₅₀ (*i.e*., concentration sufficient to inhibit 50% of the activity being targeted in the cell culture) as initially determined by the cell culture assays. Suitable experimental animals include, but are not limited to mouse, rat, guinea pigs, rabbits, pigs, monkeys and chimpanzees. As with the *in vitro* studies, initial determination is made of an effective dose of the anti-HCV compound (*e.g.,* Cₘₐₓ) and the corresponding pharmacokinetic profile. This is also carried out for the carboxylesterase inhibitors. Subsequently, lower doses of the anti-viral compounds in combination with differing doses of carboxylesterase inhibitors are used to determine a combination sufficient to achieve a similar effective level (*i.e.,* Cmax) as obtained in the absence of carboxylesterase inhibitors.

In addition, toxicity and therapeutic efficacy may also be determined by cell culture assays and/or experimental animals, typically by determining a LD₅₀ (lethal dose to 50% of the test population) and ED₅₀ (therapeutically effectiveness in 50% of the test population). This is done independently for the anti-HCV compound and the carboxylesterase inhibitor, and then for the combination. The dose ratio of toxicity and therapeutic effectiveness is the therapeutic index. Preferred are compositions and combinations of anti-HCV compounds and carboxylesterase inhibitors exhibiting high therapeutic indices.

Generally, in the case where formulations are administered to a host, anti-HCV compound and/or carboxylesterase inhibitor is given orally or as a bolus or infusion sufficient to obtain a therapeutic level in the host (*e.g.,* a specified reduction in viral count). The dosage of inhibitors will be experimentally determined to produce a specified increase in systemic of tissue exposure to the anti HCV compound. The increased exposure can be reflected in increase in plasma or tissue (*e.g.,* liver) pharmacokinetic parameters such as AUC, Cmax, elimination half life, or the decrease in clearance. These parameters are determined by measuring plasma and target tissue (*e.g.,* liver) concentrations of the anti HCV compound following pre-dosing or co-administration with carboxylesterase inhibitor. As an example, carboxyBNPP, which has an IC₅₀ of 10-50 µM, may be used in the range of about 100 mg/kg body weight, more usually in the range of about 50 mg/kg body weight of host when administered orally or intraperitoneally for a corresponding oral or bolus or infusion of an anti-HCV compound.

Dosages in the lower portion of the range and even lower dosages may be employed, where the anti-HCV compounds and carboxylesterase inhibitors are provided as a depot, such as a slow release composition comprising particles, a polymer matrix (*e.g*., a collagen matrix, carbomer, etc.) that maintains release of compounds over an extended period of time or use of a pump which continuously infuses the anti-HCV compound and carboxylesterase inhibitor over an extended period of time with a substantially continuous rate. In variations of this technique, the anti-HCV compound or the carboxylesterase inhibitor may be administered in the form of a sustained release formulation, while the other component is administered in a more rapid release formulation. These and other combinations of administering effective dosages will be apparent to those skilled in the art.

### 7. EXAMPLES

### 7.1 Example 1: Analysis of in vivo metabolism of anti-HCV compounds

Male Sprague Dawley rats were given either a single 1mg/kg (1 mL/kg) bolus intravenous dose in 70% polyethylene glycol (PEG) 400/30% saline via a tail vein, or a single 1 mg/kg (2 mL/kg) oral dose in 4% polysorbate 80/96% saline of ¹⁴C-labeled anti-HCV **II,** with radioactive dose of about 30 µCi per animal.

Groups of rats (n = 2/dose route/time point) were sacrificed at specified times from 0 to 3 h after dosing. Heparinized plasma and liver were harvested and stored at -70°C until analyzed. The plasma was assayed by liquid scintillation counting (LSC) in Beckman LS-6000LL Liquid Scintillation Counter.

Livers were homogenized in deionized water, combusted and analyzed by LSC. Portion of the homogenate was extracted by sonication with acetonitrile, centrifuged, and subjected to radioprofiling of supernatant by the Liquid Chromatography with Accurate Radioisotope Counting (LC-ARC). Agilent Series 1100 with Packard Radiomatic 150 radioactivity detector was used for analysis. Waters Spherisorb ODS1, 5 µm, 250 x 4.6 mm column was used, with mobile phase A: water/acetic acid/ammonium hydroxide (2000:20:20, v/v/v), B: acetonitrile. The radioactivity of peaks was calculated using ARC Data System software.

### 7.2 Example 2: Metabolic transformation of Compound III in vitro.

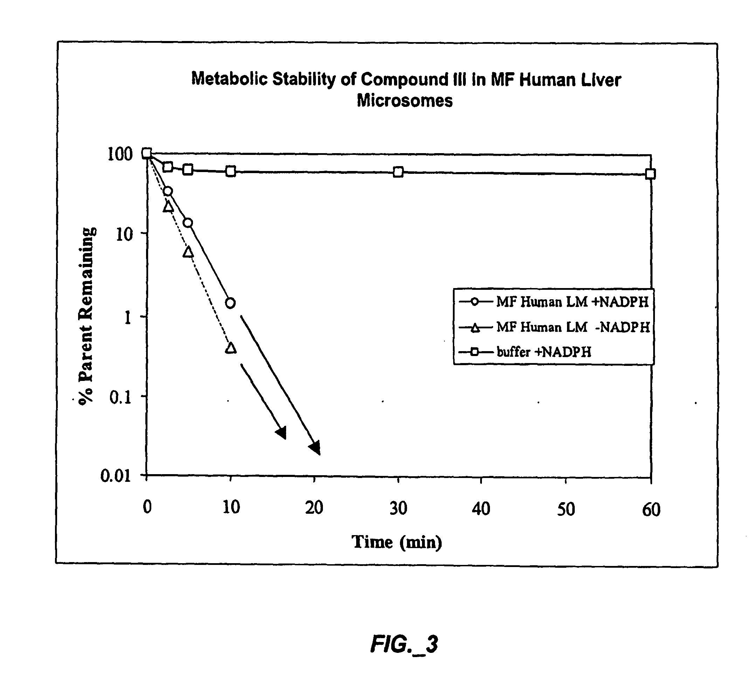

Liver microsomes prepared from human (male and/or female) or Sprague-Dawley rat (female) (Xenotech LLC, Kansas City, CS), were incubated with compound **II** (Figure 3) or **III** (Figure 4), followed by LC/MS/MS analysis of a parent compound and a primary metabolite aniline. Incubations were carried out at 37°C, at final concentrations of 0.1 M potassium phosphate buffer/0.1 mM EDTA, pH 7.4,1 µM substrate, 0.5 mg/mL microsomal protein, 0.01% DMSO, 0.3% acetonitrile, in presence or absence of 1 mM NADPH. Reactions were stopped at specified time points with acetonitrile/methanol/DMSO (50:25:25) containing an internal standard. Samples were filtered through Millipore Multiscreen filter plate and analyzed by LC/MS/MS on PE-Sciex API3000 triple quadrupole mass spectrometer, using Betasil C8 column (3x50 mm, 3µm), with mobile phase A: 0.1% formic acid in water, and B: 0.1 % formic acid in acetonitrile. Multiple reaction monitoring (MRM) in the positive ionization mode and Analyst 1.1 software were used for quantitation and data acquisition.

### 7.3 Example 3: Effect of enzyme inhibitors on transformation of anti-HCV compound.

Human male liver microsomes (Xenotech LLC, Kansas City, CS) were incubated with **III** in presence of increasing concentrations of known inhibitors of carboxylesterase, protease, and cytochrome P450 enzymes, followed by LC/MS/MS analysis of a parent compound and a primary metabolite aniline. Sodium fluoride (NaF, 5-500mM), bis(p-nitrophenyl) phosphate (BNPP, 20-500 µM), phenylmethanesulphonyl fluoride (PMSF, 5-100 µM), and proadifen (SKF 525-A, 5-100 µM) were used as inhibitors. Incubations were carried out at 37°C, at final concentrations of 0.1 M potassium phosphate buffer/0.1 mM EDTA, pH 7.4, specified concentration of inhibitor, 2 µM substrate, 0.5 mg/mL microsomal protein, 0.1 % DMSO, 0.5% acetonitrile, in absence of NADPH. Incubations were started with a substrate, following 10 min preincubation of microsomal reaction mixture with inhibitors at 37°C. Reactions were stopped at specified time points with acetonitrile/methanol/DMSO (50:25:25) containing an internal standard. Samples were filtered through Millipore Multiscreen filter plate and analyzed by LC/MS/MS on PE-Sciex API3000 triple quadrupole mass spectrometer, using Betasil C8 column (3x50 mm, 3µm), with mobile phase A: 0.1% formic acid in water, and B: 0.1% formic acid in acetonitrile. Multiple reaction monitoring (MRM) in the positive ionization mode and Analyst 1.1 software were used for quantitation and data acquisition.

### 7.4 Example 4: Inhibition of carboxyesterase isoforms and effect on metabolic transformation of anti-HCV compound.

The following purified enzymes were used for assays: carboxylesterase from porcine liver (EC 3.1.1.1, Sigma E3019); butyrylcholine esterase, equine serum (pseudocholinesterase EC 3.1.1.8, Sigma C1057); and acetylcholine esterase from electrical eel (EC 3.1.1.7, Sigma C2629).

Purified enzyme preparations (0.2-2.0 units/mL) were incubated 0-30 min at 37°C in 0.1 M potassium phosphate buffer, 0.1 mM EDTA, pH 7.4 and 0.5 mg/mL of bovine serum albumin. The substrate was delivered in 0.02% DMSO and 0.6% acetonitrile. Incubations were performed for 0-30 min at 37°C, separately for each time point. The reactions were terminated by precipitation with acetonitrile/ethanol/DMSO mixture, followed by filtering and LC/MS/MS analysis. Samples were filtered through Millipore Multiscreen filter plate and analyzed by LC/MS/MS on PE-Sciex API3000 triple quadrupole mass spectrometer, using Betasil C8 column (3 x 50 mm, 3 µm), with mobile phase A: 0.1% formic acid in water, and B: 0.1% formic acid in acetonitrile. Multiple reaction monitoring (MRM) in the positive ionization mode and Analyst 1.1 software was used for quantitation and data acquisition. Initial rates of substrate disappearance or product appearance were calculated by linear regression of 5-6 separate incubations.

### 7.5 Example 5: Inhibition of carboxylesterases on transformation of anti-HCV compound in blood.

Heparinized fresh mouse blood (1.6 mL) from Spraque-Dawley rats was added to Vacutainer Tubes: Cat # 367587, lot 2079758, approx. 2 mL volume (3 mg NaF + 6 mg disodium EDTA/tube) and spiked with **III** at 20 ng/mL blood, with concentration of NaF in blood of 0.045 mM. Samples were incubated in duplicate at 37°C up to 2 h. Control samples were incubated in eppendorf tubes without sodium fluoride. Blood concentrations of **III** and its aniline metabolite were assayed by LC/MS/MS by procedure described in Example 6.

### 7.6 Example 6: Effect of carboxylesterase inhibitors on metabolic transformation of anti-HCV compound in vivo.

Balb/c male mice were predosed intraperitoneally with 100 mg/kg (20 mg/mL) of bis(p-nitrophenyl) phosphate (BNPP) in ethanol/polyethylene glycol (PEG400) 10:40 v/v or the vehicle, one hour before dosing with **III** at 1 mg/kg (1 mg/mL) by intravenous bolus in PEG400, or at 30 mg/kg (5 mL/kg) orally in TGPS/PEG400/PG/PBS.

Blood samples were collected into heparinized Microtainer tubes, spiked with 30% BNPP solution in ethanol, 10 µl/mL blood. Livers were snap frozen in liquid nitrogen. Samples were stored at -80°C until analyzed by LC/MS/MS procedure, as described below.

The MRM mode of detection was used for LC/MS/MS analysis. Test compounds were extracted from plasma by direct protein precipitation with a mixture of acetonitrile, ethanol and dimethyl sulfoxide (DMSO) (2:1:1 v/v) and internal standard. Following vortexing and centrifugation, 10 µL of the extract was analyzed by LC-MS/MS.

Liver homogenate (20% volume in DMSO: water) was prepared using a Polytron homogenizer. Test compounds were extracted from 100 µL of the homogenized mixture as described for plasma and analyzed by LC/MS/MS.

LC/MS/MS was performed on PE-Sciex API3000 triple quadrupole mass spectrometer, using Betasil C8 column (3x50 mm, 3µm), with mobile phase A: 0.1% formic acid in water, and B: 0.1 % formic acid in acetonitrile. Multiple reaction monitoring (MRM) in the positive ionization mode and Analyst 1.1 software was used for quantitation and data acquisition.

### 7.7 Example 7: General Procedure for Preparation of anti-HCV compound microcapsules.

Typically, 3.5 g of **III** and 10 g of an anionic methacrylic polymer, either Eudragit S100, Eudragit L100 or mixtures of Eudragit L100 and Eudragit S100 are dissolved in a total of 150 mL ethanol-isopropanol-dichloromethane mixture in a volume ratio of 8:2:5. The polymer solution containing **III** is slowly introduced into a 1% polyvinyl alcohol solution (J.T. Baker, 99.0-99.8 hydrolyzed) or 1% Tween 20 solution with mechanical stirring at 250 rpm to form oil-in-water emulsion droplets. During stirring, ethanol and isopropanol diffuse into the aqueous phase. The concentration of **III** and Eudragit increases in dichloromethane and the solubility of both **III** and Eudragit decrease at the interface of the droplet. Eventually, Eudragit precipitates on the surface of the droplet and **III** being extremely hydrophobic shifts from the aqueous phase to the core or inner shell wall of droplets. After 10 minutes of stirring, the droplets of **III** and Eudragit are collected by filtration, washed with water, and dried at 50°C for 12 hours or over night.

The ethanol to isopropanol ratio is also important in forming the microcapsules described herein. Too much ethanol in the organic phase may lead to diffusion into the aqueous phase before stable emulsion droplets formed. Because isopropyl alcohol diffuses into aqueous solution at a slower rate than ethanol, emulsion droplets have more time to form. Further, the greater the solubility of a compound **III** in dichloromethane versus ethanol and isopropanol the higher the loading efficiency, since less drug will diffuse out of emulsion droplets with the alcohols.

Drug/polymer ratio, % loading, and % loading efficiency for various lots are summarized in Table 1, *infra.*

**Table 1**

| **Summary of the Eudragit Microcapsules Prepared by Solvent Diffusion Process.** | | | | |
|---|---|---|---|---|
| Lot # | Drug /Polymer Ratio | Encapsulation Polymer | % Loading | Loading Efficiency % |
| 1 | 3:10 | Eudragit S100 | 22.1 | 95.4 |
| 2 | 4.5:10 | Eudragit L100 | 31.5 | 100 |
| 3 | 3.5:10 | Eudragit S100 | 26.3 | 100 |
| 4 | 4.5:10 | Eudragit L100 | 30.2 | 97.3 |
| 5 | 3.5:10 | Eudragit S100 | 24.2 | 93.4 |
| 6 | 3.5:10 | Eudragit S100 | 24.8 | 95.7 |
| 7 | 3.5:10 | Eudragit S100 | 25.4 | 98.0 |
| 8 | 3.5:10 | Eudragit S100/L100 (1:1) | 22.7 | 87.6 |
| 9 | 3.5:10 | Eudragit S100 | 25.4 | 98.0 |

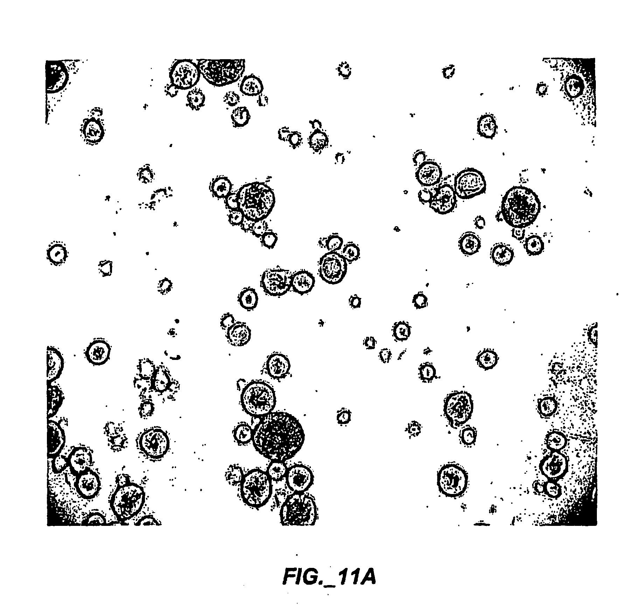

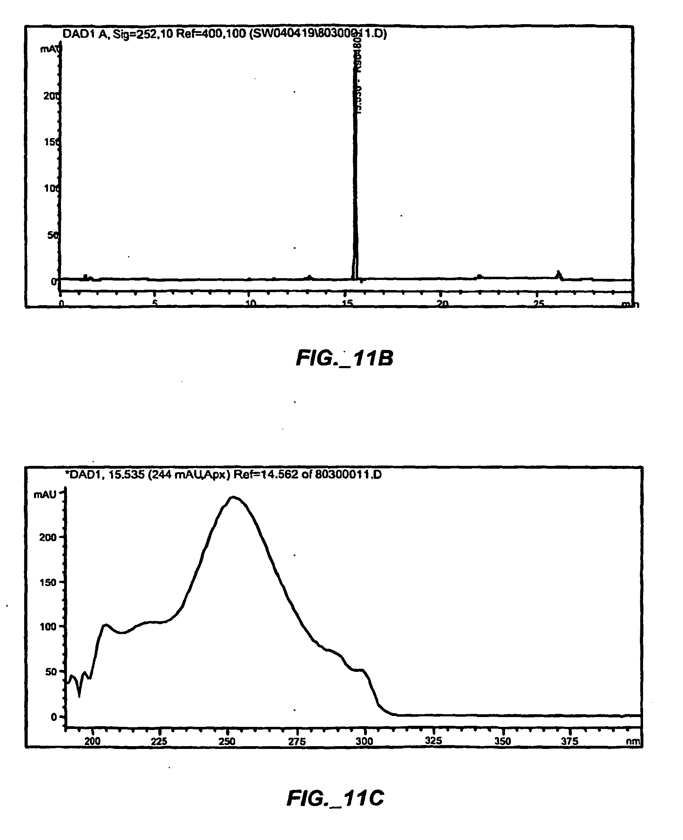

Lot 1 was characterized by polarized light microscopy (FIG. 11A) and chromatography and UV spectra (FIGS. 11B and 11C).

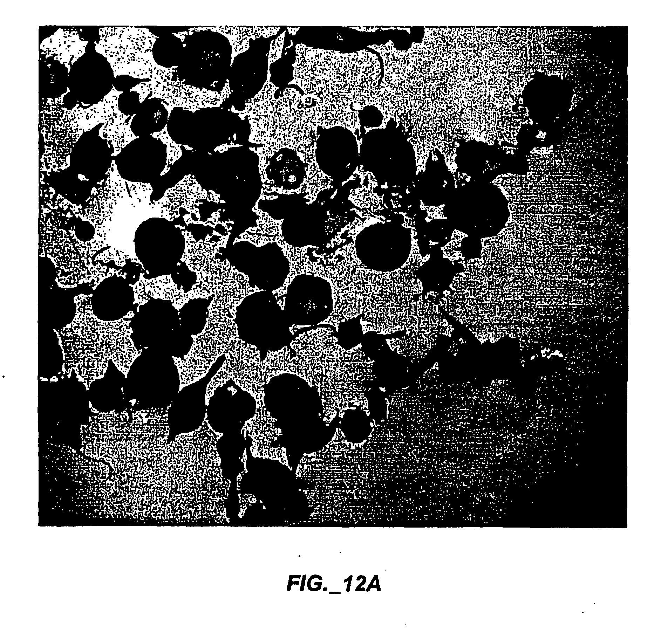

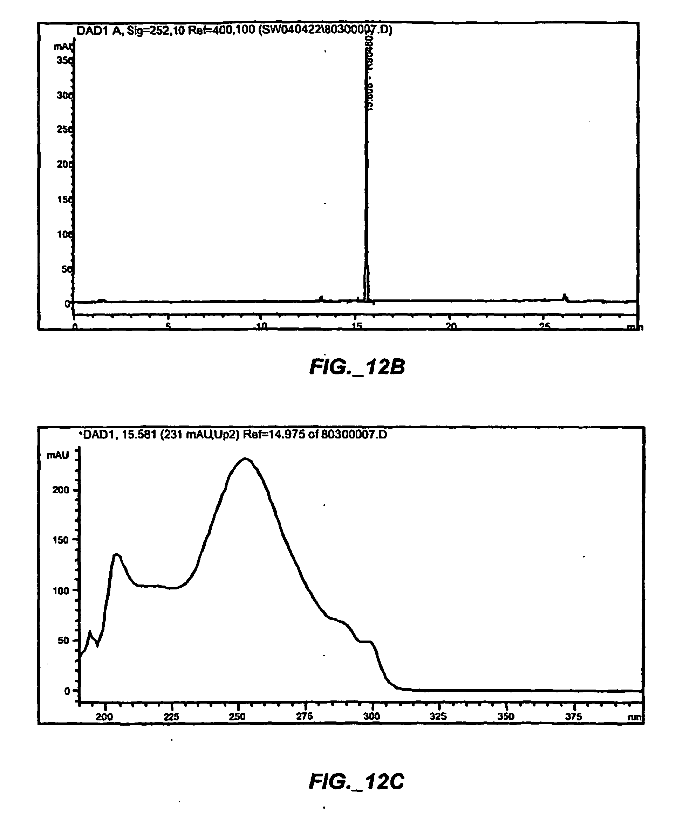

Lot 2 was characterized by polarized light microscopy (FIG. 12A) and chromatography and UV spectra (FIGS. 12B and 12C):

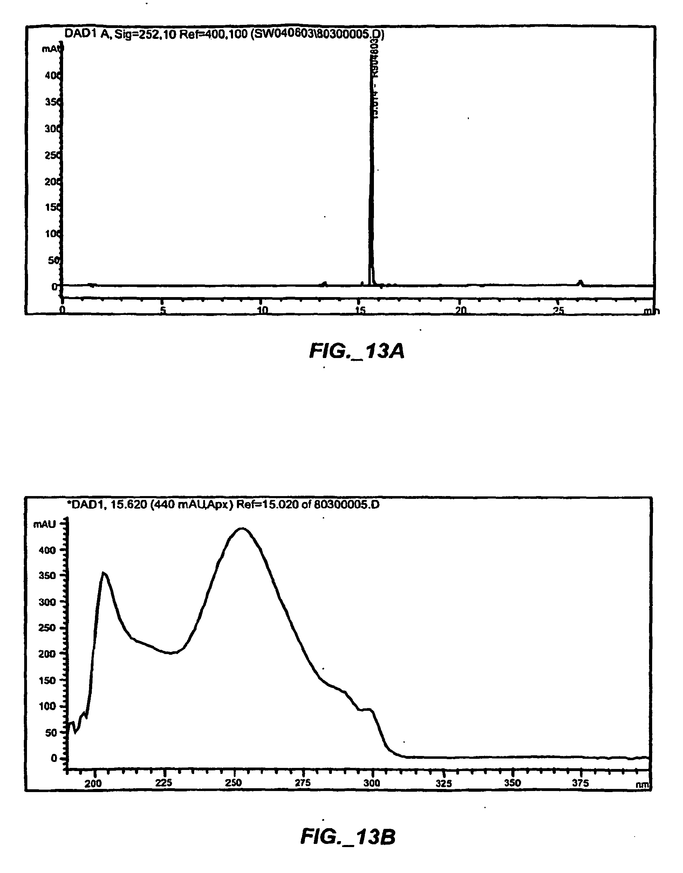

Lot 4 was characterized by chromatography and UV spectra (FIGS. 13A and 13B)).

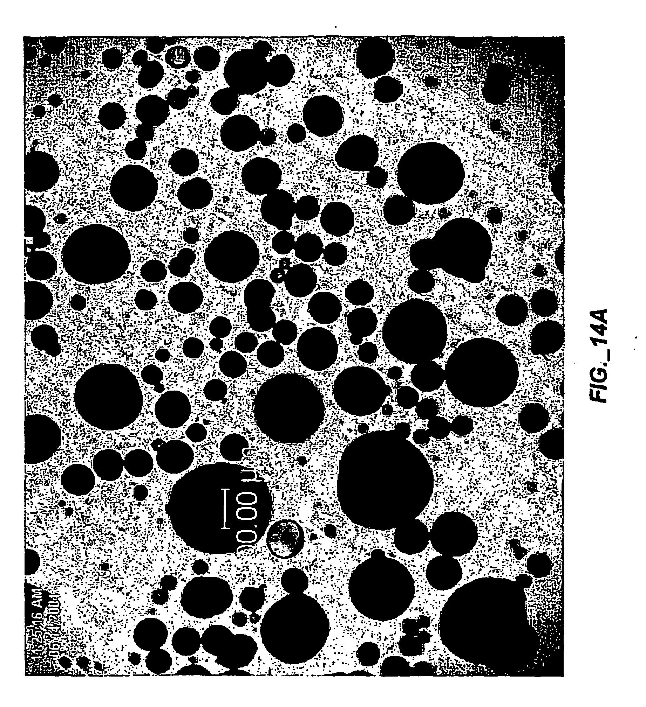

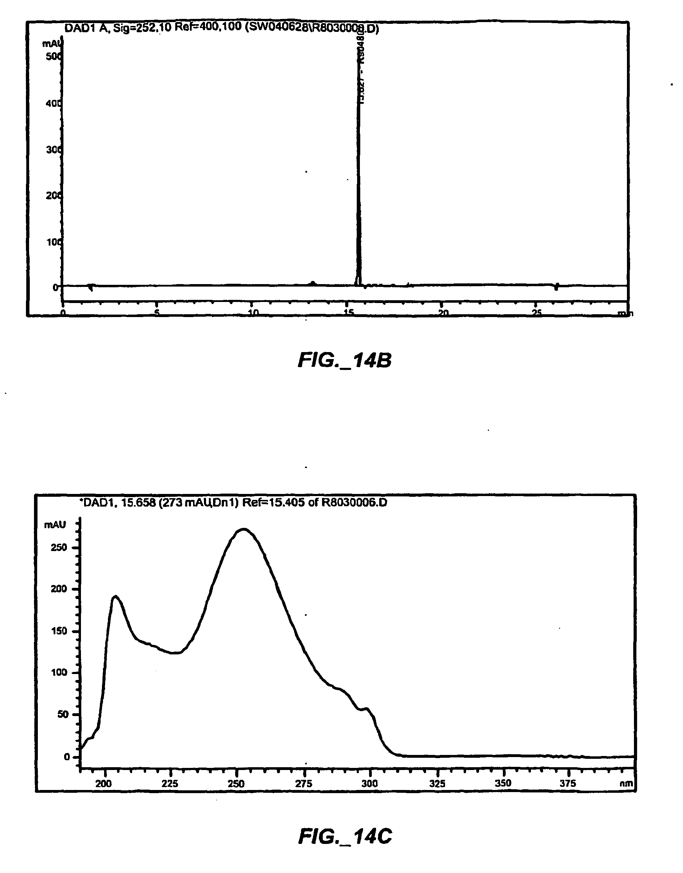

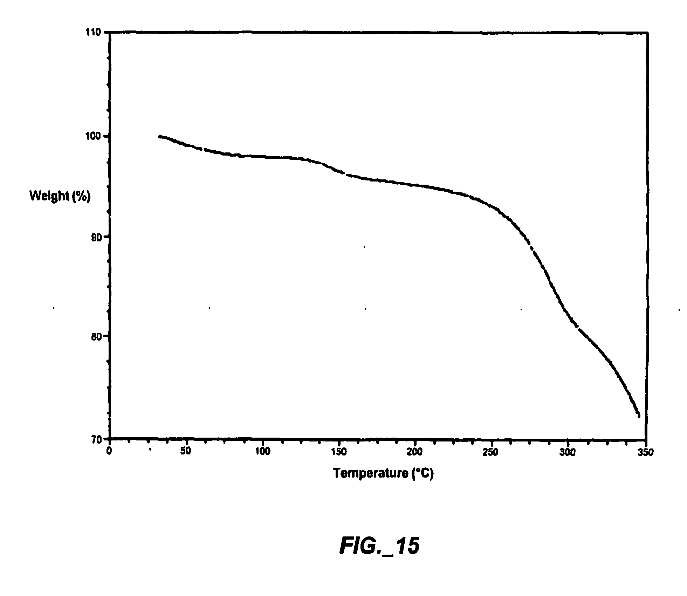

Lot 6 was characterized by polarized light microscopy (FIG. 14A), chromatography and UV spectra (FIG. 14B and 14C) and TGA profile (FIG. 15).

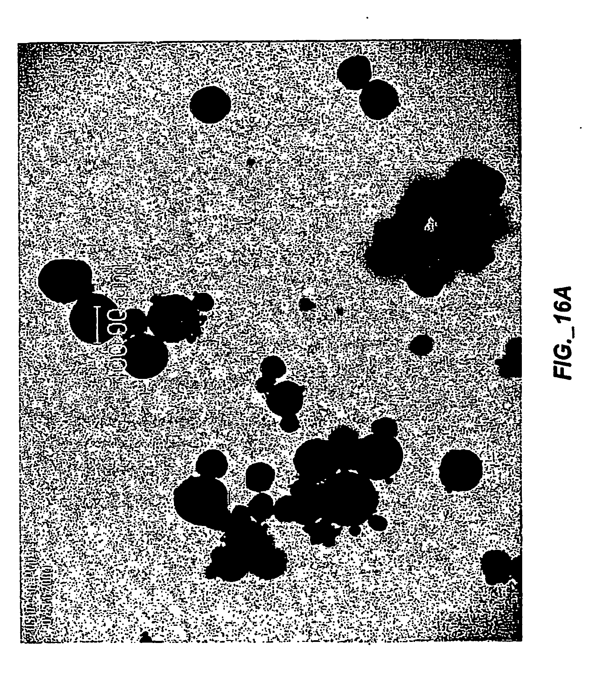

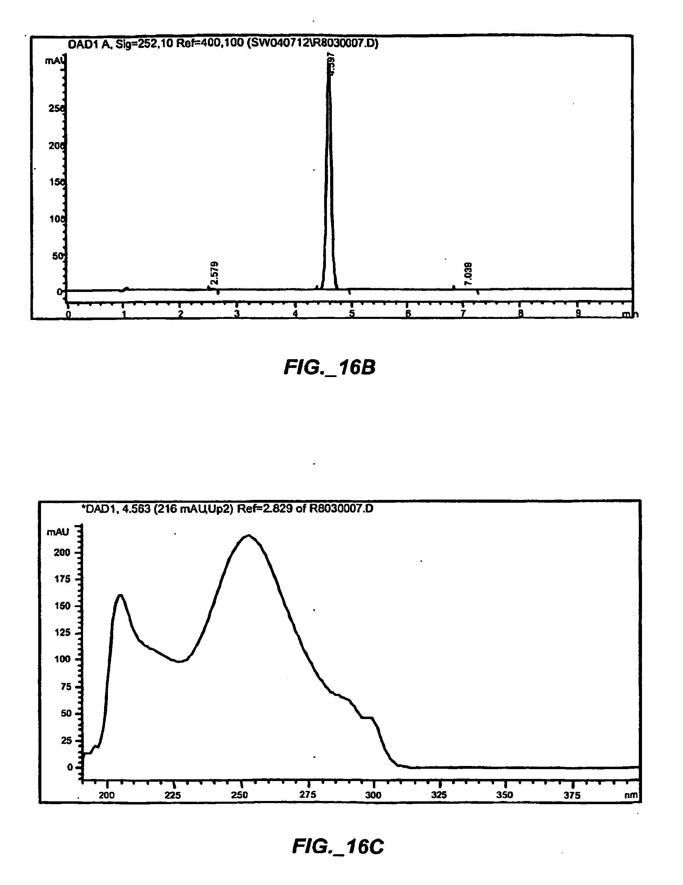

Lot 7 was characterized by polarized light microscopy (FIG. 16A) and chromatography and UV spectra (FIG. 16B and 16C).

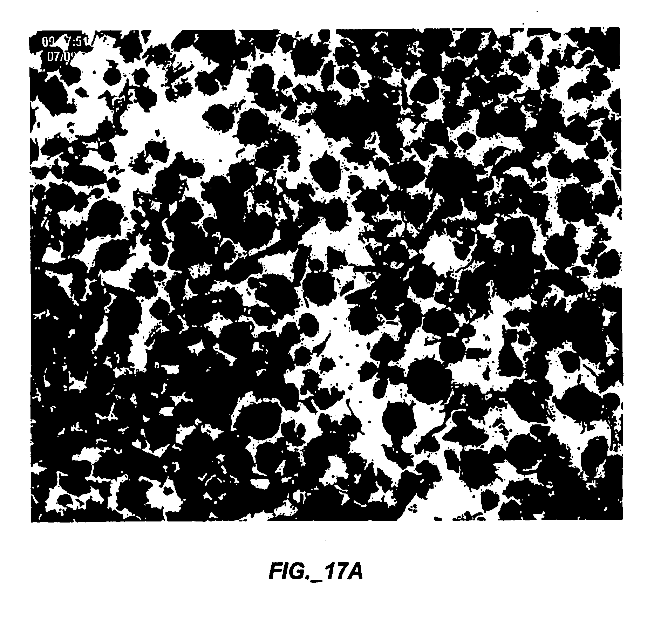

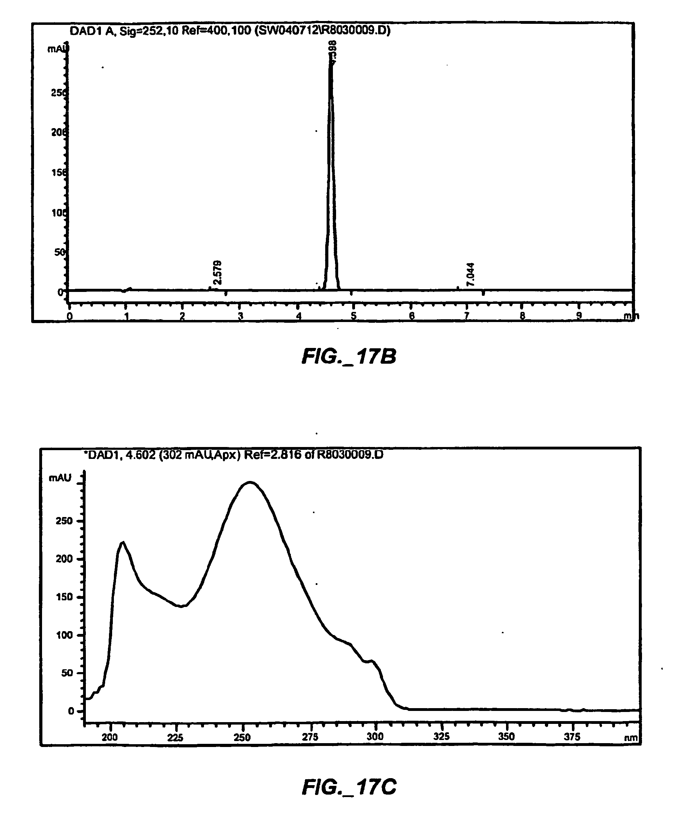

Lot 8 was characterized by polarized light microscopy (FIG. 17A) and chromatography and UV spectra (FIG. 17B and 17C).

Although the example is directed to preparing microcapsules of compound **III,** it is to be understood that the microcapsule compositions may be made for other anti-HCV compounds described herein. Further, a predictable timerelease profile of anti-HCV compounds can be attained by adjusting the thickness of the polymer layer (drug/polvmer ratio), using a different types of polymer, and controlling the speed of formation of the oil-in-water emulsion droplets.

### 7.8 Example 8: General Microencapsulation Procedure For Preparation of Ethocel MicroCapsules

Typically, 3.5 g of **III** and 10 g of Ethocel are dissolved in a total of 150 mL ethanol-isopropanol-dichloromethane mixture in a volume ratio of 8:2:5. The polymer solution containing **III** is slowly introduced into a 1% polyvinyl alcohol solution (J.T. Baker, 99.0-99.8 hydrolyzed) or 1% Tween 20 solution with mechanical stirring at 250 rpm to form oil-in-water emulsion droplets. During stirring, ethanol and isopropanol diffuse into the aqueous phase. The concentration of **III** and Ethocel increases in dichloromethane and the solubility of both **III** and Ethocel decrease at the interface of the droplet. Eventually, Ethocel precipitates on the surface of the droplet and **III** being extremely hydrophobic shifts from the aqueous phase to the core or inner shell wall of droplets. After 10 minutes of stirring, the droplets of **III** and Ethocel are collected by filtration, washed with water, and dried at 50°C for 12 hours or over night.

Drug/polymer ratio, % loading, and % loading efficiency for various lots are summarized in Table 2, *infra.*

**Table 2**

| **Summary of the Ethocel Microcapsules Prepared by Solvent Diffusion Process.** | | | | |
|---|---|---|---|---|
| Lot # | Drug /Polymer Ratio | Encapsulation Polymer | % Loading | Loading Efficiency % |
| 10 | 3:10 | Ethocel 20CPS | 21.4 | 92.7 |
| 11 | 1:1 | Ethocel 20 CPS | 48.4 | 96.8 |

### 7.9 Example 9: Morphology, DSC and X-ray study of Eudragit Microcapsules

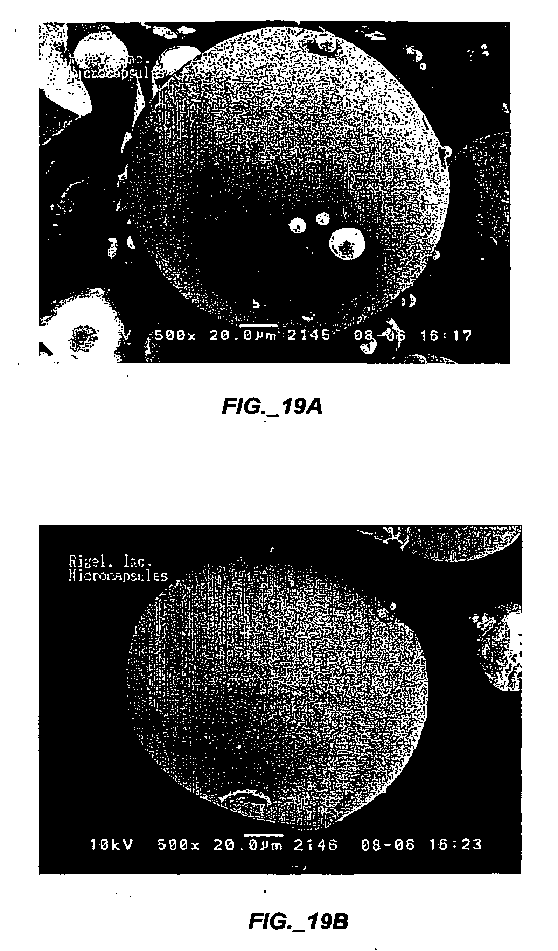

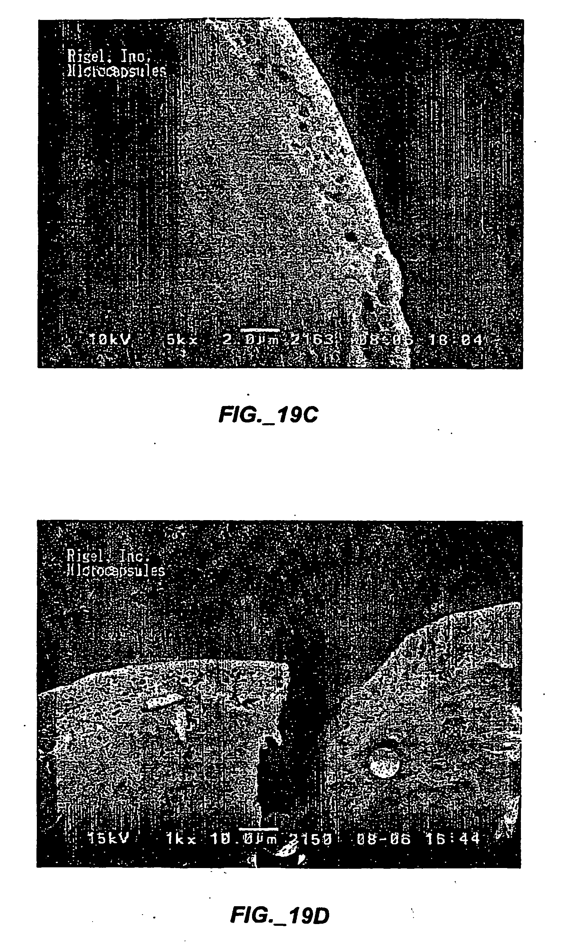

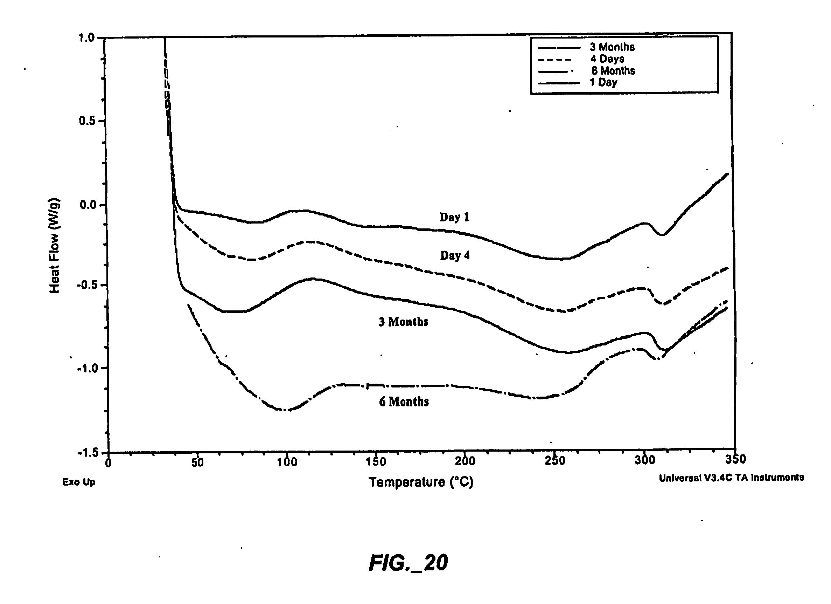

FIGS. 19A and 19B illustrate that the outer surface of the microcapsule is smooth. FIGS 19C and 19D illustrate the porous internal surface and porous shell; the pore size in the shell is less than about 1 µm and the shell thickness varies from about 2 µm to 15 µm. DSC study (FIG. 20) of the microcapsules demonstrated that no crystals of **III** exist (absence of sharp peak at about 190°C) when the microcapsule is first prepared and for up to six months when stored at room temperature. Similarly, the measured X-ray diffraction pattern of the microcapsules demonstrated the absence of any crystalline material in the sample.

### 7.10 Example 10: Dissolution of Eudragit Microcapsules

Lots 3 and 4 (see Examples 1) were tested for drug release at 100 rpm at 37°C at pH 6.8 and 7.4. FIG. 22A shows that the dissolution rate of Eudragit S microcapsules at pH 7.4 is much faster than at pH 6.8. FIG. 22B shows that the dissolution rate of Eudragit L microcapsules at pH 6.8. is much faster than that of Eudragit S microcapsules. Accordingly, Eudragit L microcapsules may be better candidates for small intestine and duodenum targeted delivery.

### 7-11 Example 11: Preparation of Tablets of Eudragit Microcapsules

Microcapsules of Lot 7 (see Example 1) were formulated into tablets using Avicel PH 101, Ac-Di-Sol, stearic acid, and 316 Past-Flo Lactose as excipients. Table 3 summarizes the physical properties of these tablets. The microcapsules remained intact through out the direct compression process.

**Table 3**

| | mg/tab | Wt% | Total wt (g) |
|---|---|---|---|
| Lot 7 microcapsules | 160 | 26.7 | 3.52 |
| Ethocel 20cps | 90 | 15 | 1.98 |
| Stearic acid | 60 | 10 | 1.32 |
| Avicel PH 101 | 130 | 21.7 | 2.86 |
| AC-Di-Sol | 30 | 5 | 0.66 |
| 316 Fast-Flo Lactose | 130 | 21.6 | 2.86 |
| Total | 600 | 100 | 13.2 |
| 1. Compressed at 800 lbs using CRAVER Hydraulic Press with 0.3150"X0.6299" Oval Shape punches and die | | | |
| 2. Weight of 20 tablets = 12.04g or 602.4 mg per tablet. | | | |
| 3. Average Tablet Thickness: 0.296". | | | |
| 4. Tablet Hardness: 7.2 kilogram | | | |
| 5. Disintegration time in 0.1N HCl was around 10 minutes. | | | |

### 7.12 Example 12: Preparation of Capsules for Eudragit Microcapsules.

Microencapsulates of **III** prepared as described above were packed into HPMC capsules and used for pharmacokinetics (PK) studies in dogs, monkeys and chimpanzees. Capsules contained an equivalent of about 40 mg **III** per capsule. The following amounts of microcapsule preparations from various lots were used for making the capsules used in the PK studies:
(a) Size one HPMC microcapsules (0.49 ml body volume) were packed with approximately 128 mg of **III**/Eudragit L100 microcapsules, Lot 2, and submitted for PK study in dogs;
(b) Size one HPMC capsules were packed with approximately 154 mg each with **III**/Eudragit S100 microcapsules, Lot 3, and submitted for PK study in monkeys;
(c) Size one HPMC capsules were packed with approximately 133 mg each with **III**/Eudragit L100 microcapsules, Lot 4, and submitted for PK study in monkeys; and
(d) **III**/Eudragit S 100 microcapsules, Lot 6, were submitted as powder in bottle for oral PK and efficacy studies in chimpanzees.

### 7.13 Example 13: Pharmacokinetic (PK) Studies in Cyano Monkeys

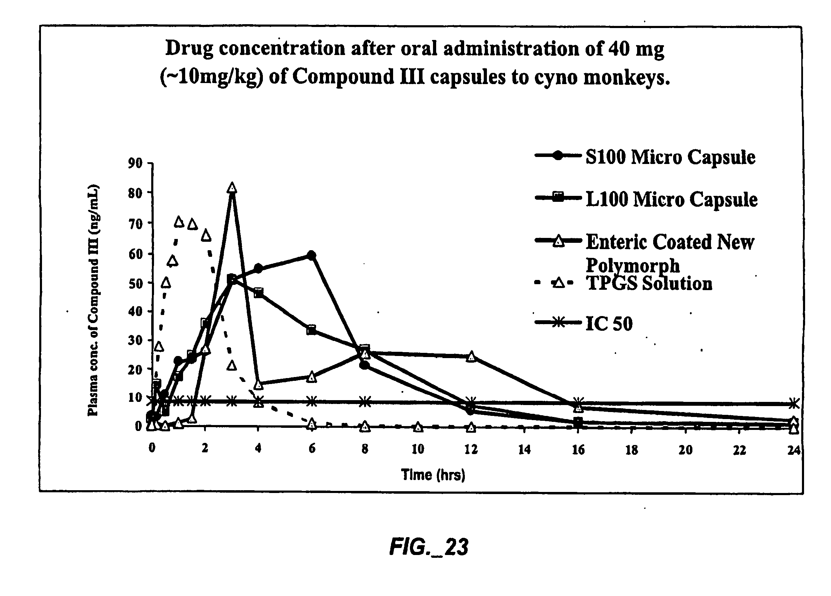

Microcapsules containing **III** were packed into HPMC capsules or glass vials and used for pharmacokinetics studies in monkeys. As is shown in FIG. 23 both the Eudragit S100 and L100 microcapsules provide superior pharmacokinetic profile over TPGS solution (2 fold increase) in cyno monkeys. The concentration of **III** also lasted 10 hours above the IC50 in replicon assays.

The foregoing descriptions of specific embodiments in the present disclosure have been presented for purposes of illustration and description. They are not intended to be exhaustive or to limit the invention to the precise forms disclosed, and obviously many modifications and variations are possible in light of the above teachings.

## Claims

1. A composition for use as a pharmaceutical comprising a compound that comprises a haloalkylamide moiety, and means for protecting the compound from metabolism by a hydrolase.

2. The composition of claim 1 in which the haloalkylamide moiety is a gem-dichloroacetamide moiety.

3. The composition of claim 1 in which the compound is a compound according to the structural formula (I): 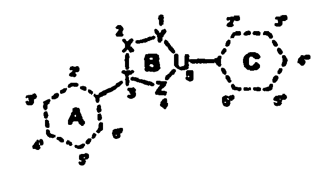 including the salts, hydrates, and solvates thereof, wherein:
the "A" ring comprises a substituted phenyl or a substituted pyridyl;
the "B" ring comprises a saturated, unsaturated or aromatic 5-membered ring that optionally includes a heteroatom at one or more of positions X, Y, and Z, each heteroatom being independently selected from NH, N, O and S, with the proviso that X and Y are not both O;
the "C" ring comprises a phenyl or pyridyl that is substituted at the 3" or 5" position with a haloalkylamide moiety of the formula -NR¹¹C(O)R¹², where R¹¹ is hydrogen or alkyl and R¹² is a haloalkyl, a dihalomethyl, or a dichloromethyl, and which may optionally include one or more of the same or different additional unillustrated substituents.

4. The composition of claim 3 in which R¹¹ is H and R¹² is dichloromethyl.

5. The composition of claim 3 in which "A" and "C" rings are phenyl.

6. The composition of claim 3 in which one or both of the "A" and "C" rings are a pyridyl.

7. The composition of claim 3 in which the compound is

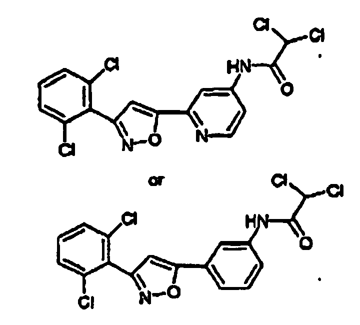

8. The composition of claim 1 in which the means comprises a carboxylesterase inhibitor.

9. The composition of claim 8 in which the carboxylesterase inhibitor inhibits a carboxylesterase isozyme.

10. The composition of claim 9 in which the carboxylesterase isozyme is a human carboxylesterase.

11. The composition of claim 8 in which the carboxylesterase inhibitor inhibits an intestinal carboxylesterase.

12. The composition of claim 8 in which the carboxylesterase inhibitor inhibits a liver carboxylesterase.

13. The composition of claim 8 in which the carboxylesterase inhibitor is selected from a trifluorakylketone compound, an organophosphate compound, an aminoacridine compound, and mixtures thereof.

14. The composition of claim 8 in which the carboxylesterease inhibitor is a mixed ester compound.

15. The composition of claim 8 in which the carboxylesterase inhibitor comprises a mixture of carboxylesterase inhibitors.

16. The composition of claim 15 in which each carboxylesterase inhibitor of the mixture inhibits a different carboxylesterase isozyme.

17. The composition of any preceeding claim further comprising a pharmaceutically acceptable vehicle.

18. A composition according to any one of claims 1-17 for use in medicine.

19. Use of a compound that comprises a haloalkylamide moiety and a carboxylesterase inhibitor for the preparation of a medicament for treating a subject in need thereof.

20. Use of a compound that comprises a haloalkylamide moiety for the preparation of a medicament that further comprises a carboxylesterase inhibitor for treating a patient in need thereof.

21. Use of a compound that comprises a carboxylesterase inhibitor for the preparation of a medicament that further comprises a haloalkylamide moiety, for treating a patient in need thereof.

22. The use of any one of claims 19 to 21 in which the compound is a compound comprising the structural formula (I): 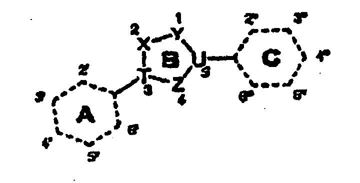 including the salts, hydrates and solvates thereof, wherein
the "A" ring comprises a substituted phenyl or or a substituted pyridyl;
the "B" ring comprises a saturated, unsaturated or aromatic 5-membered ring that optionally includes a heteroatom at one or more of positions X, Y, and Z, each heteroatom being independently selected from NH, N,O and S, with the proviso that X and Y are not both O ;
the "C" ring comprises a phenyl or pyridyl that is substituted at the 3" or 5" position with a haloalkylamide moiety of the formula-NR¹¹C (O)R¹² wherein R¹¹ is hydrogen or alkyl and R¹² is a haloalkyl, a dihalomethyl, or a dichloromethyl, and which may optionally include one or more of the same or different additional unillustrated substituents.

23. The use of any one of claims 19 to 21 in which the administration of the compound is by a first route of administration and administration of the carboxylesterase inhibitor is by a second route of administration.

24. The use of claim 23 in which the first and the second routes of administration are the same.

25. The use of claim 23 in which the first and second routes of administration are different.

26. The use of any one of claims 19 to 21 in which the compound and the carboxylesterase inhibitor are administered sequentially.

27. The use of any one of claims 19 to 21 in which the compound and the carboxylesterase inhibitor are administered simultaneously.

28. The use of any one of claims 19 to 21 in which the carboxylesterase inhibitor inhibits a carboxylesterase isozyme.

29. The use of any one of claims 19 to 21 in which the carboxylesterase inhibitor inhibits an intestinal carboxylesterase.

30. The use of any one of claims 19 to 21 in which the carboxylesterase inhibitor inhibits a liver carboxylesterase.

31. The use of any one of claims 19 to 21 in which the carboxylesterase inhibitor is selected from atrifluoromethylketone compound, an organophosphate compound, an aminoacridine compound, and mixtures thereof.

32. The use of any one of claims 19 to 21 in which the carboxyleterase inhibitor is a mixed ester compound.

33. The use of claim 32 in which the carboxylesterase inhibitor comprises a mixture of carboxylesterase inhibitors.

34. The use of Claim 33 in which each carboxylesterase inhibitor of the mixture inhibits a different carboxylesterase isozyme.

35. Use of a composition according to any one of claims 1-17 for the preparation of a medicament for modulating the bioavailability of an anti-viral compound.

36. Use of a carboxylesterase inhibitor for the preparation of a medicament for modulating the bioavailability of an anti-viral compound that comprises a haloalkylamide moiety in a subject.

37. Use of a carboxylesterase inhibtor for the preparation of a medicament for modulating the bioavailability of an anti-viral compound that comprises a haloalkylamide moiety in a subject, comprising adjunctively administering to the subject the compound and a carboxylesterase inhibitor.

38. Use of a composition according to any one of claims 1-17 for the preparation of a medicament for inhibiting the metabolism of an anti-viral compound in a subject.

39. Use of a carboxylesterase inhibitor for the preparation of a medicament for inhibiting the metabolism of an anti-viral compound that comprises a haloalkylamide moiety in a subject.

40. Products containing a compound that comprises a haloalkylamide moiety and a carboxylesterase inhibitor as an adjunctive preparation for separate, simultaneous or sequential use.

41. A composition comprising a composition according to any one of claims 1-18 that is microencapsulated.

42. The composition according to claim 41 wherein the microencapsulation is with a composition comprising an enteric polymer.

43. The composition according to claim 42 wherein the microencapsulation is with a composition comprising an enteric polymer selected from methacrylic acid/methyl methacrylate copolymer, methacrylic acid/ethyl acrylate copolymer, methacrylic acid/methyl acrylate/methyl methacrylate copolymer polyacrylic acid, polyacrylate, polyacrylamide, ethyl cellulose, hydroxypropyl cellulose, hydroxypropylmethyl cellulose phthalate, carboxymethyl cellulose, polyvinyl acetate phthalate, carboxymethylethyl cellulose, shellac, acrylic resins, acetate succinate, cellulose acetate phthalate, cellulose acetate trimellitate, or combinations thereof.

## Patentansprüche

1. Zusammensetzung für die Verwendung als ein pharmazeutisches Mittel, welche eine Verbindung, die eine Halogenalkylamidgruppe umfaßt, und Mittel zum Schützen der Verbindung gegenüber dem Metabolismus durch eine Hydrolase umfaßt.

2. Zusammensetzung nach Anspruch 1, bei der die Halogenalkylamidgruppe eine geminale Dichloracetamidgruppe ist.

3. Zusammensetzung nach Anspruch 1, bei der die Verbindung eine Verbindung der Strukturformel (I) ist: 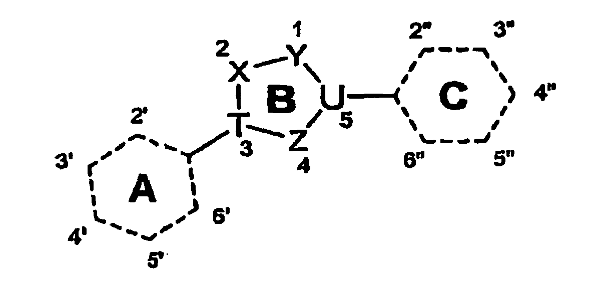 einschließlich die Salze, Hydrate und Solvate davon, wobei:
der "A"-Ring ein substituiertes Phenyl oder ein substituiertes Pyridyl umfaßt,
der "B"-Ring einen gesättigten, ungesättigten oder aromatischen 5-gliedrigen Ring umfaßt, der wahlweise ein Heteroatom in einer oder mehreren der Positionen X, Y und Z einschließt, wobei jedes Heteroatom unabhängig ausgewählt ist unter NH, N, O und S, mit der Maßgabe, daß X und Y nicht beide O sind,
der "C"-Ring ein Phenyl oder Pyridyl umfaßt, welches in der 3"- oder 5"-Position mit einer Halogenalkylamidgruppe der Formel -NR¹¹C (O) R¹² substituiert ist, wobei R¹¹ Wasserstoff oder Alkyl ist und R¹² ein Halogenalkyl, ein Dihalogenmethyl oder ein Dichlormethyl ist, und welches wahlweise einen oder mehrere der gleichen oder verschiedenen zusätzlichen nicht dargestellten Substituenten einschließen kann.

4. Zusammensetzung nach Anspruch 3, bei der R¹¹ H und R¹² Dichlormethyl ist.

5. Zusammensetzung nach Anspruch 3, bei der die "A"- und "C"- Ringe Phenyl sind.

6. Zusammensetzung nach Anspruch 3, bei der einer oder beide der "A"- und "C"-Ringe Pyridyl sind.

7. Zusammensetzung nach Anspruch 3, bei der die Verbindung 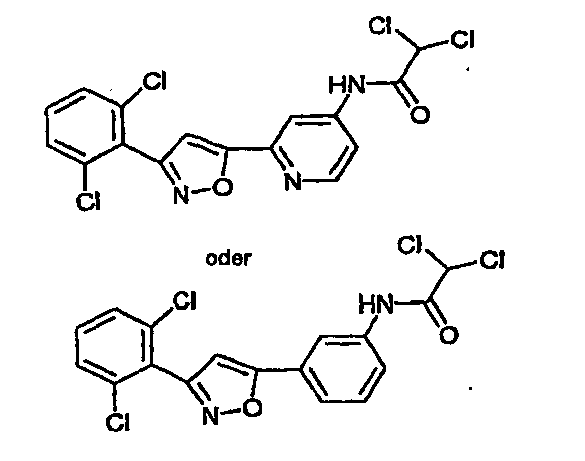 ist.

8. Zusammensetzung nach Anspruch 1, bei der das Mittel einen Carboxylesteraseinhibitor umfaßt.

9. Zusammensetzung nach Anspruch 8, bei der der Carboxylesteraseinhibitor ein Carboxylesteraseisozym hemmt.

10. Zusammensetzung nach Anspruch 9, bei der das Carboxylesteraseisozym eine humane Carboxylesterase ist.

11. Zusammensetzung nach Anspruch 8, bei der der Carboxylesteraseinhibitor eine intestinale Carboxylesterase hemmt.

12. Zusammensetzung nach Anspruch 8, bei der der Carboxylesteraseinhibitor eine Leber-Carboxylesterase hemmt.

13. Zusammensetzung nach Anspruch 8, bei der der Carboxylesteraseinhibitor ausgewählt ist unter einer Trifluoralkylketonverbindung, einer organischen Phosphatverbindung, einer Aminoakridinverbindung und Gemischen davon.

14. Zusammensetzung nach Anspruch 8, bei der der Carboxylesteraseinhibltor eine gemischte Esterverbindung ist.

15. Zusammensetzung nach Anspruch 8, bei der der Carboxylesteraseinhibitor ein Gemisch von Carboxylesteraseinhibitoren umfaßt.

16. Zusammensetzung nach Anspruch 15, bei der jeder Carboxylesteraseinhibitor des Gemischs ein anderes Carboxylesteraseisozym hemmt.

17. Zusammensetzung nach Anspruch 1, die weiterhin c) einen pharmazeutisch verträglichen Träger umfaßt.

18. Zusammensetzung nach einem der Ansprüche 1 bis 17 für die Verwendung in der Medizin.

19. Verwendung einer Verbindung, die eine Halogenalkylamidgruppe und einen Carboxylesteraseinhibitor umfaßt, für die Herstellung eines Arzneimittels zur Behandlung eines Patienten, der dessen bedarf.

20. Verwendung einer Verbindung, die eine Halogenalkylamidgruppe umfaßt, für die Herstellung eines Arzneimittels, das weiterhin einen Carboxylesteraseinhibitor umfaßt, für die Behandlung eines Patienten, der dessen bedarf.

21. Verwendung einer Verbindung, die einen Carboxylesteraseinhibitor umfaßt, für die Herstellung eines Arzneimittels, das weiterhin eine Halogenalkylamidgruppe umfaßt, für die Behandlung eines Patienten, der dessen bedarf.

22. Verwendung nach einem der Ansprüche 19 bis 21, bei der die Verbindung eine Verbindung ist, die die Strukturformel (I) umfaßt: 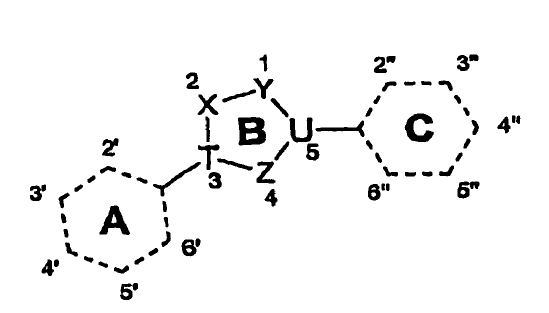 einschließlich die Salze, Hydrate und Solvate davon, wobei
der "A"-Ring ein substituiertes Phenyl oder ein substituiertes Pyridyl umfaßt,
der "B"-Ring einen gesättigten, ungesättigten oder aromatischen 5-gliedrigen Ring umfaßt, der wahlweise ein Heteroatom in einer oder mehreren der Positionen X, Y und Z einschließt, wobei jedes Heteroatom unabhängig ausgewählt ist unter NH, N, O und S, mit der Maßgabe, daß X und Y nicht beide O sind,
der "C"-Ring ein Phenyl oder Pyridyl umfaßt, welches in der 3"- oder 5"-Position mit einer Halogenalkylamidgruppe der Formel -NR¹¹C (O) R¹² substituiert ist, wobei R¹¹ Wasserstoff oder Alkyl ist und R¹² ein Halogenalkyl, ein Dihalogenmethyl oder ein Dichlormethyl ist, und welches wahlweise einen oder mehrere der gleichen oder verschiedenen zusätzlichen nicht dargestellten Substituenten einschließen kann.

23. Verfahren nach einem der Ansprüche 19 bis 21, bei dem die Verabreichung der Verbindung über einen ersten Verabreichungsweg erfolgt und die Verabreichung des Carboxylesteraseinhibitors über eine zweiten Verabreichungsweg erfolgt.

24. Verwendung nach Anspruch 23, bei der der erste und der zweite Verabreichungsweg gleich sind.

25. Verwendung nach Anspruch 23, bei der der erste und der zweite Verabreichungsweg verschieden sind.

26. Verwendung nach den Ansprüchen 19 bis 21, bei der die Verbindung und der Carboxylesteraseinhibitor nacheinander verabreicht werden.

27. Verwendung nach den Ansprüchen 19 bis 21, bei der die Verbindung und der Carboxylesteraseinhibitor gleichzeitig verabreicht werden.

28. Verwendung nach den Ansprüchen 19 bis 21, bei der der Carboxylesteraseinhibitor ein Carboxylesteraseisozym hemmt.

29. Verwendung nach den Ansprüchen 19 bis 21, bei der der Carboxylesteraseinhibitor eine intestinale Carboxylesterase hemmt.

30. Verwendung nach den Ansprüchen 19 bis 21, bei der der Carboxylesteraseinhibitor eine Leber-Carboxylesterase hemmt.

31. Verwendung nach den Ansprüchen 19 bis 21, bei der der Carboxylesteraseinhibitor ausgewählt ist unter einer Trifluormethylketonverbindung, einer organischen Phosphatverbindung, einer Aminoakridinverbindung und Gemischen davon.

32. Verwendung nach den Ansprüchen 19 bis 21, bei der der Carboxylesteraseinhibitor eine Gemischte-Ester-Verbindung ist.

33. Verwendung nach Anspruch 32, bei der der Carboxylesteraseinhibitor ein Gemisch von Carboxylesteraseinhibitoren umfaßt.

34. Verwendung nach Anspruch 33, bei der jeder Carboxylesteraseinhibitor des Gemischs ein anderes Carboxylesteraseisozym hemmt.

35. Verwendung einer Zusammensetzung nach einem der Ansprüche 1 bis 17 für die Herstellung eines Arzneimittels zum Modulieren der Bioverfügbarkeit einer anti-viralen Verbindung.

36. Verwendung eines Carboxylesteraseinhibitors zur Herstellung eines Arzneimittels zum Modulieren der Bioverfügbarkeit einer anti-viralen Verbindung, welche eine Halogenalkylamidgruppe umfaßt, in einem Patienten.

37. Verwendung eines Carboxylesteraseinhibitors zur Herstellung eines Arzneimittels zum Modulieren der Bioverfügbarkeit einer anti-viralen Verbindung, die eine Halogenalkylamidgruppe umfaßt, in einem Patienten, wobei man dem Patienten die Verbindung und einen Carboxylesteraseinhibitor gemeinsam verabreicht.

38. Verwendung einer Zusammensetzung nach einem der Ansprüche 1 bis 17 für die Herstellung eines Arzneimittels zum Hemmen des Metabolismus einer anti-viralen Verbindung in einem Patienten.

39. Verwendung eines Carboxylesteraseinhibitors für die Herstellung eines Arzneimittels zum Hemmen des Metabolismus einer anti-viralen Verbindung, die eine Halogenalkylamidgruppe umfaßt, in einem Patienten.

40. Produkte, die als eine adjunktive Zubereitung für die separate, gleichzeitige oder aufeinanderfolgende Verwendung eine Verbindung, welche eine Halogenalkylamidgruppe umfaßt, und einen Carboxylesteraseinhibitor enthalten.

41. Zusammensetzung mit einer Zusammensetzung nach einem der Ansprüche 1 bis 18, welche mikroverkapselt ist.

42. Zusammensetzung nach Anspruch 41, wobei die Mikroverkapselung mit einer Zusammensetzung erfolgt, die ein magensaftresistentes Polymer umfaßt.

43. Zusammensetzung nach Anspruch 42, wobei die Mikroverkapselung mit einer Zusammensetzung erfolgt, die ein magensaftresistentes Polymer umfaßt, welches ausgewählt ist unter Methacrylsäure/Methylmethacrylat-Copolymer, Methacrylsäure/Ethylacrylat-Copolymer, Methacrylsäure/Methyacrylat/Methylmethacrylat-Copolymer, Polyacrylsäure, Polyacrylat, Polyacrylamid, Ethylcellulose, Hydroxypropylcellulose, Hydroxypropylmethylcellulosephthalat, Carboxymethylcellulose, Polyvinylacetatphthalat, Carboxymethylethylcellulose, Schellack, Acrylharze, Acetatsuccinat, Celluloseacetatphthalat, Celluloseacetattrimellitat oder Kombinationen davon.

## Revendications

1. Composition destinée à être utilisée comme agent pharmaceutique, comprenant un composé qui comprend un groupement halogénalkylamide, et un moyen pour protéger le composé contre le métabolisme par une hydrolase.

2. Composition suivant la revendication 1, dans laquelle le groupement halogénalkylamide est un groupement gem-dichloracétamide.

3. Composition suivant la revendication 1, dans laquelle le composé est un composé répondant à la formule structurale (I) : 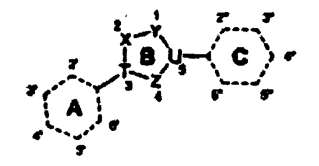 ainsi que ses sels, hydrates et produits de solvatation, formule dans laquelle :
le noyau "A" comprend un groupe phényle substitué ou un groupe pyridyle substitué ;
le noyau "B" comprend un noyau pentagonal saturé, insaturé ou aromatique qui comprend facultativement un hétéroatome à une ou plusieurs positions des X, Y et Z, chaque hétéroatome étant choisi indépendamment entre NH, N, O et S, sous réserve que X et Y ne représentent pas l'un et l'autre O ;
le noyau "C" comprend un groupe phényle ou pyridyle qui est substitué en position 3" ou 5" avec un groupement halogénalkylamide de formule -NR¹¹C(O)R¹², dans laquelle R¹¹ représente un atome d'hydrogène ou un groupe alkyle et R¹² représente un groupe halogénalkyle, dihalogénométhyle ou dichlorométhyle, et qui peut comprendre facultativement un ou plusieurs des substituants non illustrés supplémentaires identiques ou différents.

4. Composition suivant la revendication 3, dans laquelle R¹¹ représente H et R¹² représente un groupe dichlorométhyle.

5. Composition suivant la revendication 3, dans laquelle les noyaux "A" et "C" sont des noyaux phényle.

6. Composition suivant la revendication 3, dans laquelle l'un des ou les deux noyaux "A" et "C" sont des noyaux pyridyle.

7. Composition suivant la revendication 3, dans laquelle le composé répond à la formule 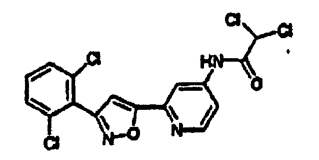 ou

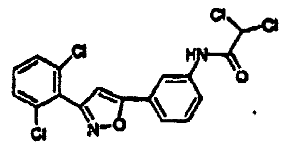

8. Composition suivant la revendication 1, dans laquelle le moyen comprend un inhibiteur de carboxylestérase.

9. Composition suivant la revendication 8, dans laquelle l'inhibiteur de carboxylestérase inhibe une isozyme de carboxylestérase.

10. Composition suivant la revendication 9, dans laquelle l'isozyme de carboxylestérase est une carboxylestérase humaine.

11. Composition suivant la revendication 8, dans laquelle l'inhibiteur de carboxylestérase inhibe une carboxylestérase intestinale.

12. Composition suivant la revendication 8, dans laquelle l'inhibiteur de carboxylestérase inhibe une carboxylestérase hépatique.

13. Composition suivant la revendication 8, dans laquelle l'inhibiteur de carboxylestérase est choisi entre une trifluoralkylcétone, un organophosphate, une aminoacridine et leurs mélanges.

14. Composition suivant la revendication 8, dans laquelle l'inhibiteur de carboxylestérase est un ester mixte.

15. Composition suivant la revendication 8, dans laquelle l'inhibiteur de carboxylestérase comprend un mélange d'inhibiteurs de carboxylestérase.

16. Composition suivant la revendication 15, dans laquelle chaque inhibiteur de carboxylestérase du mélange inhibe une isozyme de carboxylestérase différente.

17. Composition suivant la revendication 1, comprenant en outre c) un véhicule pharmaceutiquement acceptable.

18. Composition suivant l'une quelconque des revendications 1 à 17, destinée à être utilisée en médecine.

19. Utilisation d'un composé qui comprend un groupement halogénalkylamide et d'un inhibiteur de carboxylestérase pour la préparation d'un médicament destiné au traitement d'un sujet en ayant besoin.

20. Utilisation d'un composé qui comprend un groupement halogénalkylamide pour la préparation d'un médicament qui comprend en outre un inhibiteur de carboxylestérase pour le traitement d'un sujet en ayant besoin.

21. Utilisation d'un composé qui comprend un inhibiteur de carboxylestérase pour la préparation du médicament qui comprend en outre un inhibiteur de carboxylestérase, pour le traitement d'un patient en ayant besoin.

22. Utilisation suivant l'une quelconque des revendications 19 à 21, dans lequel le composé est un composé comprenant la formule structurale (I) : 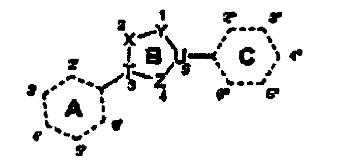 ainsi que ses sels, hydrates et produits de solvatation, formule dans laquelle :
le noyau "A" comprend un groupe phényle substitué ou un groupe pyridyle substitué ;
le noyau "B" comprend un noyau pentagonal saturé, insaturé ou aromatique qui comprend facultativement un hétéroatome à une ou plusieurs des positions X, Y et Z, chaque hétéroatome étant choisi indépendamment entre NH, N, O et S, sous réserve que X et Y ne représentent pas l'un et l'autre O ;
le noyau "C" comprend un groupe phényle ou pyridyle qui est substitué en position 3" ou 5" avec un groupement halogénalkylamide de formule -NR¹¹C(O)R¹², dans laquelle R¹¹ représente un atome d'hydrogène ou un groupe alkyle et R¹² représente un groupe halogénalkyle, dihalogénométhyle ou dichlorométhyle, et qui peut comprendre facultativement un ou plusieurs des substituants non illustrés supplémentaires identiques ou différents.

23. Utilisation suivant l'une quelconque des revendications 19 à 21, dans lequel l'administration du composé est effectuée par une première voie d'administration et l'administration de l'inhibiteur de carboxylestérase est effectuée par une seconde voie d'administration.

24. Utilisation suivant la revendication 23, dans laquelle les première et seconde voies d'administration sont identiques.

25. Utilisation suivant la revendication 23, dans laquelle les première et seconde voies d'administration sont différentes.

26. Utilisation suivant les revendications 19 à 21, dans laquelle le composé et l'inhibiteur de carboxylestérase sont administrés de manière séquentielle.

27. Utilisation suivant les revendications 19 à 21, dans laquelle le composé et l'inhibiteur de carboxylestérase sont administrés de manière simultanée.

28. Utilisation suivant les revendications 19 à 21, dans laquelle l'inhibiteur de carboxylestérase inhibe une isozyme de carboxylestérase.

29. Utilisation suivant les revendications 19 à 21, dans laquelle l'inhibiteur de carboxylestérase inhibe une carboxylestérase intestinale.

30. Utilisation suivant les revendications 19 à 21, dans laquelle l'inhibiteur de carboxylestérase inhibe une carboxylestérase hépatique.

31. Utilisation suivant les revendications 19 à 21, dans laquelle l'inhibiteur de carboxylestérase est choisi entre une trifluorométhylcétone, un organophosphate, une aminoacridine et leurs mélanges.

32. Utilisation suivant les revendications 19 à 21, dans laquelle l'inhibiteur de carboxylestérase est un ester mixte.

33. Utilisation suivant la revendication 32, dans laquelle l'inhibiteur de carboxylestérase comprend un mélange d'inhibiteurs de carboxylestérase.

34. Utilisation suivant la revendication 33, dans laquelle chaque inhibiteur de carboxylestérase du mélange inhibe une isozyme de carboxylestérase différente.

35. Utilisation d'une composition suivant l'une quelconque des revendications 1 à 17 pour la préparation d'un médicament destiné à moduler la biodisponibilité d'un composé antiviral.

36. Utilisation d'un inhibiteur de carboxylestérase pour la préparation d'un médicament destiné à moduler la biodisponibilité d'un composé antiviral qui comprend un groupement halogénalkylamide chez un sujet.

37. Utilisation d'un inhibiteur de carboxylestérase pour la préparation d'un médicament destiné à moduler la biodisponibilité d'un composé antiviral qui comprend un groupement halogénalkylamide chez un sujet, comprenant l'administration d'appoint au sujet du composé et d'un inhibiteur de carboxylestérase.

38. Utilisation d'une composition suivant l'une quelconque des revendications 1 à 17 pour la préparation d'un médicament destiné à inhiber le métabolisme d'un composé antiviral chez un sujet.

39. Utilisation d'un inhibiteur de carboxylestérase pour la préparation d'un médicament destiné à inhiber le métabolisme d'un composé antiviral qui comprend un groupement halogénalkylamide chez un sujet.

40. Produits contenant un composé qui comprend un groupement halogénalkylamide et un inhibiteur de carboxylestérase comme préparation d'appoint pour une utilisation de manière séparée, simultanée ou séquentielle.

41. Composition comprenant une composition suivant l'une quelconque des revendications 1 à 18 qui est micro-encapsulée;

42. Composition suivant la revendication 41, dans laquelle la micro-encapsulation est effectuée avec une composition comprenant un polymère entérique.

43. Composition suivant la revendication 42, dans laquelle la micro-encapsulation est effectuée avec une composition comprenant un polymère entérique choisi entre un copolymère acide méthacrylique/méthacrylate de méthyle, un copolymère acide méthacrylique/acrylate d'éthyle, un copolymère acide méthacrylique/acrylate de méthyle/méthacrylate de méthyle, l'acide polyacrylique, un polyacrylate, le polyacrylamide, l'éthylcellulose, l'hydroxypropylcellulose, le phtalate d'hydroxypropylméthylcellulose, la carboxyméthylcellulose, l'acétophtalate de polyvinyle, la carboxyméthyléthylcellulose, la gomme laque, des résines acryliques, un acétosuccinate, l'acétophtalate de cellulose, l'acétotrimellitate de cellulose et leurs associations.
